# EUROPEAN PATENT SPECIFICATION

(11) **EP 4 237 043 B1**
(45) Date of publication and mention of the grant of the patent: **11.02.2026**
(21) Application number: 21802651.6
(22) Date of filing: 29.10.2021
(51) Int. Cl.: A61M 5/20, A61M 5/31, A61F 9/00, A61M 5/315, A61M 5/50

(54) **ADMINISTRATION DEVICE FOR IN PARTICULAR INTRAVITREAL ADMINISTRATION OF A FLUID**
VERABREICHUNGSVORRICHTUNG ZUR INSBESONDERE INTRAVITREALEN VERABREICHUNG EINES FLUIDS
DISPOSITIF D'ADMINISTRATION, EN PARTICULIER POUR L'ADMINISTRATION INTRAVITRÉENNE D'UN FLUIDE

(30) Priority: 30.10.2020 EP 20205001
(43) Date of publication of application: 06.09.2023
(73) Proprietor: Boehringer Ingelheim International GmbH, 55216 Ingelheim am Rhein (DE)
(72) Inventor: ROTH, Axel, 55216 Ingelheim am Rhein (DE); SCHUY, Steffen, 55216 Ingelheim am Rhein (DE); CULLIS WATSON, Benjamin Harry, Bristol BS7 9DU (GB); LABAT-ROCHECOUSTE, Andrew Guy, Bristol BS31 1AU (GB); QATTAN, Ahlam Jawdat, Barnoldswick, Lancashire BB18 6AA (GB); HAYTON, Paul Graham, Bristol BS6 5AU (GB); FOGG, Oliver David, Bristol BS2 0EH (GB)
(74) Representative: Von Rohr Patentanwälte Partnerschaft mbB
(86) International application number: PCT/EP2021/080118
(87) International publication number: WO 2022/090463

(56) References cited:
- US-A1- 2011 184 351
- US-A1- 2013 079 718
- US-A1- 2013 324 965
- US-A1- 2014 371 670
- US-A1- 2016 220 327
- US-A1- 2017 087 304
- US-A1- 2019 001 065

## Description

The present invention relates to an administration device for preferably ophthalmic, in particular intravitreal, administration of a fluid according to the preamble of claim 1.

The present invention relates generally to the field of administration/delivery devices for pharmaceutical administration/delivery of a fluid, in particular a drug, to a patient, i.e. a human or animal.

The administration device according to the present invention is preferably adapted to deliver, in particular inject, a drug (directly) into the eye, in particular the vitreous, anterior and/or posterior chambers of the eye, of a human or animal. Thus, the present invention in particular relates to administration/delivery devices for ophthalmic, in particular intravitreal, injection of a fluid, in particular a drug. However, the administration device according to the present invention can also be used for administration/injection of a fluid, in particular a drug, into other parts of the human or animal body.

WO 2014/005728 A1 relates to a small volume syringe suitable for ophthalmic injections, wherein the syringe is pre-filled with a drug and comprises a body, a stopper and a plunger. The plunger comprises a plunger contact surface arranged to contact the stopper such that the plunger can be used to force the stopper towards an outlet, thereby delivering a fluid contained within the syringe.

US 2006/0069350 A1 relates to a medical syringe injector pen with a syringe and a housing for accommodating the syringe. The pen comprises a drive mechanism and an actuation member for releasing the drive mechanism and actuating the device to deliver a fluid from the syringe to a user.

US 2010/0231102 A1 relates to an intravitreal injection device including an injection assembly adapted to receive a syringe. The injection assembly is adapted to automatically transfer the syringe into the patient's eye and then to dispense the fluid contained in the syringe into the eye.

US 2013/0324965 A1 discloses a one-time use device to deliver preservative-free follicle stimulating hormone solution, wherein the device includes a needle covered by a sliding needle shield which covers the needle in all modes of the device. The device can be placed into a ready-to-use position in four of fewer user steps.

US 2019/0001065 A1 disclosed a medicament delivery device configured to provide a variable single dose of medicament. The device includes a main body and a syringe arranged in the main body, wherein the syringe comprises a medicament. The medicament delivery device further includes a removable cap and a plunger rod operatively arranged to eject the medicament through a delivery member i.e. an injection needle attached to the syringe.

US 2014/0371670 A1 discloses a medicament delivery device for a reconstituted medicament comprising a proximal housing part, a distal housing part, a medicament delivery drive unit and actuation means, wherein the medicament delivery device unit is mounted in said distal housing part and wherein the actuation means further include an activation member which is displaceable between an inactive position wherein the actuator is prevented from engaging the medicament delivery drive unit and an active position wherein the actuators are able to engage the medicament delivery drive, whereby the actuator is capable of interacting with said medicament delivery drive unit to perform a delivery of the reconstituted medicament only in the active position of the activation member and in the second position of the medicament delivery drive unit.

US 2017/0087304 A1 discloses a medicament delivery device having a housing with a distal and a proximal end, the housing being adapted to receive a medicament container with a delivery member or with a connectable delivery member for delivery of a medicament. The device also has a drive mechanism arranged to act on a plunger rod that in turn is arranged to act on the medicament container for providing automatic delivery of the medicament and a hold-release mechanism interactively connected to the drive mechanism for releasing the drive mechanism from a biased position.

US 2016/0220327 A1 discloses a dispensing pen which removably retains a single use capsule containing tooth whitening compound and removably retains disposable tooth whitening applicators.

US 2011/0184351 A1 discloses a medical delivery device comprising a housing, a medicament container arranged to be placed in the housing, a stopper movable within said container, a medicament dispensing means attachable to said housing and to said container through which the medicament can be expelled. The device further comprises a power unit comprising a cover, a spring arrange within said cover, and a pressure member having a pressure pad in contact with a stopper, a guide rod extending into the spring and an annular ledge in contact with the edges of the spring.

US 2013/0079718 A1 discloses an automatic injection device including a housing, a syringe, a plunger and a syringe carrier. The housing includes a barrel which includes an elongated window to allow viewing of contents inside the housing. The syringe is disposed within the housing and has a reservoir. The plunger is at least partially disposed within the syringe and includes a visual indicator.

The object of the present invention is to provide an improved - in particular solely mechanical - administration/delivery device which is user-friendly and/or easy, intuitive and/or safe to use and/or minimizes or at least reduces the risk of misapplication, misuse, contamination, incorrect and/or imprecise dosing, and/or of the delivery/administration of air bubbles and/or minimizes or at least reduces the requirements and/or complexity of or within the manufacturing processes of the administration device.

The above object is achieved by an administration device according to claim 1. Advantageous further developments are subject of the sub claims.

The administration/delivery device, hereinafter referred to as administration device, according to the present invention is preferably adapted to (immovably) hold an in particular cylindrical container for a fluid and/or containing the fluid, in particular a drug.

Preferably, the administration device comprises the container and/or a plunger/piston/stopper movably arranged within the container, in particular such that by an axial movement of the plunger/piston/stopper, hereinafter referred to as stopper, towards the outlet of the container the fluid contained in the container is dispensed.

Further, the administration device comprises a rack/chassis, hereinafter referred to as rack, preferably wherein the container is or can be in particular immovably held by and/or at least partially arranged within the rack.

The administration device comprises an actuation mechanism for actuating the administration device and/or moving the stopper within the container, in particular for so-called priming of the administration device prior to administering the fluid and/or for dispensing the fluid out of the container, as will be explained in the following.

The administration device, in particular its actuation mechanism, comprises a main unit - in particular directly and/or axially - acting on the stopper, a preferably preloaded/compressed drive spring - in particular directly and/or axially - acting on the main unit, a first actuation part and a second actuation part, wherein the first actuation part and the second actuation part are movably attached to the rack and/or wherein the main unit is in particular axially movable relative to and/or arranged rack by actuating the first actuation part and/or the second actuation part, in particular in order to prime the administration device and/or to dispense the fluid out of the container.

The actuation mechanism of the administration device is preferably configured to (axially) move the main unit, the stopper and/or the drive spring from an initial/unactuated position to an administration/ready-to-use/primed position during priming, prior to administration and/or by actuating the first actuation part, and/or from the administration/ready-to-use/primed position to a final/actuated/end-of-use position after priming, during (subsequent) administration and/or by at least partially releasing the drive spring and/or by actuating the second actuation part.

According to one aspect of the present invention, the administration device, in particular the actuation mechanism, comprises a leadscrew, wherein the first actuation part and the main unit (directly) movably interact with one another and/or are (directly) movably connected to one another by means of the leadscrew and/or wherein the first actuation part is (manually) rotatable, in particular relative to the rack and/or the container, in order to axially move the main unit in particular relative to the rack and/or the container, preferably such that rotation of the first actuation part moves the main unit and/or the (compressed) drive spring axially, towards the stopper and/or out of the initial/unactuated position into the administration/ready-to-use/primed position.

Preferably, the first actuation part forms an axial end and/or a (rotatable) cap of the administration device that can be manually actuated, in particular rotated, in order to axially move the main unit.

By actuating, in particular rotating, the first actuation part and, thereby, moving the main unit, the stopper and/or the drive spring, it is possible to - at least partially manually - prime the administration device and/or to push out air/gas contained within the container and/or a needle attached thereto.

In this way, an easy, intuitive and user-friendly priming of the administration device is achieved.

In the context of the present invention, the term "prime" / "priming" preferably refers to the (necessary) preparation of the administration device (immediately) prior to administration of the fluid contained in the administration device, in particular its container.

Preferably, during priming, the main unit is (axially) moved such that and/or until the main unit, in particular a plunger/connecting rod thereof, abuts the stopper.

Mostly preferred, during priming, the main unit is (axially) moved such that the main unit, in particular a plunger/connecting rod thereof, moves/pushes the stopper - in particular towards an outlet - and/or such that a (small) part of the fluid is dispensed and/or air, i.e. air bubbles contained in the fluid, container and/or a needle attached thereto, is/are removed, in particular pushed out.

The administration device is preferably held vertically during priming, in particular such that the outlet of the container and/or a needle attached thereto points upwards and/or that air can be at least partially pushed out of the container.

Preferably, the volume contained in the container after priming corresponds to a desired/predefined dose to be administered to a patient/user.

Typically, the volume of the fluid contained in the (pre-filled) container exceeds the volume of the dose to be administered so that air contained in the fluid, container and/or the needle attached thereto can be removed at least partially by priming without effecting the dose to be administered.

However, priming of the administration device does not imply that the container is actually filled with a fluid and/or that a part of the fluid is dispensed. In particular, the actuation mechanism, mostly preferred the first actuation part, can also be actuated even if the container is empty and/or without moving and/or acting on the stopper.

In other words, "prime" / "priming" in particular means a movement of the main unit, the stopper and/or the drive spring from the initial/unactuated position into a defined position, hereinafter referred to as the administration/ready-to-use/primed position, in particular prior to administering the fluid and/or by actuating the first actuation part.

Accordingly, all actions performed prior to administering the fluid and/or actuating the second actuation part are to be considered as priming and/or the priming process.

Priming is preferably done/completed solely by (completely) actuating, in particular rotating, the first actuation part.

In the context of the present invention, the term "administer" / "administration" preferably relates to the (actual) delivery/dispensing of the fluid from the administration device, preferably its container, mostly preferred through an injection needle attached thereto.

While it is preferred that the administration device according to the present invention is used for administering a fluid, in particular a drug, directly to the body of a patient, e.g. by using an injection needle and/or by injecting a fluid directly into the eye of a patient, in particular the vitreous body, the term "administer" / "administration" also refers to delivery/dispensing the fluid without a patient being present.

Preferably, "administer" / "administration" means and/or includes a movement of the main unit and/or the stopper from the administration/ready-to-use/primed position to a final/actuated/end-of-use position, in particular after priming and/or after (completely) actuating the first actuation part.

The initial/unactuated position, hereinafter referred to as the initial position, is preferably the (axial) position of the main unit, the stopper and/or the drive spring relative to the rack and/or container before priming of the administration device and/or actuating the first actuation part and/or in the initial state of the administration device.

In the initial state of the administration device and/or when the main unit, the stopper and/or the drive spring are/is in the initial position, the first actuation part and the second actuation part are unactuated and/or the drive spring is (most) tensioned.

It is preferably not possible to (directly) actuate the second actuation part and/or to (directly) administer the fluid in the initial state of the administration device and/or when the main unit, the stopper and/or the drive spring are/is in the initial position.

The main unit, the stopper and/or the drive spring can be moved out of the initial position and/or from the initial position to the administration/ready-to-use/primed position in particular solely by actuating the first actuation part.

The administration/ready-to-use/primed position, hereinafter referred to as administration position, is preferably the (axial) position of the main unit, the stopper and/or the drive spring relative to the rack and/or the container (immediately) after priming and/or (immediately) after (completely) actuating, in particular rotating, the first actuation part and/or (immediately) before administration is carried out and/or in the administration/ready-to-use/primed state of the administration device, hereinafter referred to as administration state.

In the administration state of the administration device and/or when the main unit, the stopper and/or the drive spring are/is in the administration position, the first actuation part is (completely) actuated and the second actuation part is (still) unactuated.

In the administration state/position, the main unit, the stopper and/or the drive spring are/is arranged closer to an outlet of the administration device, in particular the container, and/or further away from the first actuation part compared to the initial state/position. In particular, the main unit and/or the stopper are/is inserted further into the container in the administration state/position compared to the initial state/position.

In the administration state of the administration device and/or when the main unit, the stopper and/or the drive spring are/is in the administration position, the main unit, in particular a plunger/connecting rod thereof, abuts the stopper and/or the administration device is ready-to-use, the second actuation part can be (directly) actuated and/or the fluid can be administered to a patient without performing further steps apart from actuating the second actuation part.

It is preferably not possible to (again) actuate and/or reverse the actuation of the first actuation part in the administration state/position.

The final/actuated/end-of-use position, hereinafter referred to as final position, is preferably the (axial) position of the main unit and/or stopper relative to the rack and/or the container (immediately) after administration and/or after (completely) actuating the second actuation part and/or in the final/actuated/end-of-use state of the administration device, hereinafter referred to as final state.

In the final state of the administration device and/or when the main unit and/or the stopper are/is in the final position, the administration has been carried out, the second actuation part is (completely) actuated and/or the drive spring / its spring force/energy is at least partially released/expanded.

In the final state/position, the main unit and/or the stopper are/is arranged closer to the outlet of the administration device and/or further away from the first actuation part compared to the initial and/or administration state/position.

Preferably, the main unit and/or the stopper are/is inserted further into the container in the final state/position compared to the initial and/or administration state/position.

In particular, the drive spring is less tensioned in the final state/position than in the administration and/or initial state/position.

However, it is preferred that - for dose accuracy - the drive spring is still tensioned, in particular compressed, in the final state/position.

In particular, no further fluid can be dispensed/administered in the final state and/or after the final position is reached.

It is preferably not possible to (again) actuate and/or reverse the actuation of the second actuation part in the final state and/or after the final position is reached.

As already mentioned, the first actuation part is preferably actuated by (manually) rotating the first actuation part relative to the rack and/or the container, mostly preferred about and/or at least essentially transverse/perpendicular to the main axis of the administration device and/or to the direction of injection.

The second actuation part is preferably actuated by (manually) depressing the second actuation relative to the rack and/or the container, mostly preferred about and/or at least essentially transverse/perpendicular to the main axis of the administration device and/or to the direction of injection.

In this way, a possible impact of the actuation of the first actuation part and/or the second actuation part on the injection side is minimized or at least reduced.

According to another aspect of the present invention which can be realized also independently, the drive spring / its spring force/energy of the actuation mechanism is at least partially releasable/expanded by actuating, in particular rotating, the first actuation part and/or during, in particular at the end of, priming and/or prior to administration and at least partially (further) releasable/expandable by actuating, in particular depressing, the second actuation part and/or after priming and/or during administering.

In other words, the first actuation part is configured to at least partially release, in particular expand, the drive spring / its spring force/energy in a first step and the second actuation part is configured to at least partially (further) release, in particular expand, the drive spring / its spring force/energy in a second/subsequent step, preferably wherein the second actuation part can only be actuated after the first actuation part has been (completely) actuated and/or in the administration state of the administration device and/or in the administration position of the main unit and/or the stopper.

Preferably, the first actuation part is configured to move the main unit, the stopper and/or the drive spring - in particular together and/or as a unit - from the initial position to a releasing/disengaging position in which the drive spring / its spring force/energy is at least partially released/expanded such that the further/subsequent movement of the main unit and/or the stopper is supported and/or completed by means of the drive spring, in particular its spring force, and/or such that the main unit and/or the stopper are/is further/subsequently moved from the releasing position to the administration position - in particular automatically and/or solely - by means of the drive spring, in particular its spring force.

In other words, the main unit, the stopper and/or the drive spring are/is first moved in a first step and/or from the initial position to the releasing/disengaging position manually and/or (solely) by actuating, in particular rotating, the first actuation part and then moved in a second step and/or from the releasing/disengaging position to the administration position automatically and/or - in particular solely - by partially releasing the drive spring, i.e. by the spring force/energy of the drive spring.

In particular, priming and/or the end of the priming process is preferably solely completed by the drive spring, i.e. its spring force.

The releasing/disengaging position, hereinafter referred to as releasing position, is preferably an (axial) position of the main unit, the stopper and/or the drive spring relative to the rack and/or the container during priming and/or between the initial position and the administration position and/or (immediately) before reaching the administration position, the administration state and/or completing priming.

In the releasing position and/or during priming and/or (immediately) before reaching the administration position, the drive spring / its spring force/energy is (automatically) at least partially released/expanded, in particular such that the subsequent movement of the main unit and/or the stopper towards the administration position and/or the transition of the administration device from the initial state to the administration state is completed and/or supported by means of the drive spring.

Thus, according to one aspect of the invention, priming and/or the movement of the main unit and/or the stopper during priming and/or from the initial position to the administration position is carried out in a semi-manual / semi-automatic manner and/or both manually, i.e. by actuating, in particular rotating, the first actuation part, and automatically, i.e. by at least partially expanding/releasing the drive spring / its spring force.

Preferably, the drive spring / its spring force/energy is at least partially ex-pandd/released and/or less tensioned in the administration state and/or when the main unit, the stopper and/or the drive spring are in the administration position compared to the initial state and/or when the main unit, the stopper and/or the drive spring are in the initial position.

The benefits of the semi-manual / semi-automatic priming are an improved usability of the administration device and a better control of the priming process compared to fully automated priming.

The drive spring, in particular its spring force, facilitates the transition from the initial state to the administration state of the administration device. Further, releasing/expanding of the drive spring / its spring force/energy during priming clearly indicates to a user/practitioner that the manual part of the priming process has been correctly completed.

Further, the critical components, i.e. the components mechanically interacting with one another during administration, are already moved/pushed into position during priming by means of the drive spring so that the shock caused by expanding/releasing the drive spring / its spring force/energy for the first time occurs during (the less critical) priming and before (the more critical) administering of the fluid. In this way, the risk of incorrect and/or imprecise dosing and the impact of dimensional tolerances during administration is reduced.

According to another aspect of the present invention that can be realized also independently, the first actuation part can be actuated in order to in particular axially move the main unit, the stopper and/or the drive spring - in particular together and/or as one unit - from the initial position to the releasing position, preferably wherein the actuation mechanism comprises a securing element configured to prevent expanding/releasing of the drive spring / its spring force/energy during movement of the main unit and/or the drive spring from the initial position to the releasing position and to in particular automatically at least partially expand/release the drive spring / its spring force/energy in the releasing position and/or when the releasing position is reached, preferably such that the administration position is reached automatically and/or solely by means of the drive spring, i.e. its spring force, and/or without further actuating the first actuation part, as already explained.

In this way, corresponding advantages can be achieved.

Preferably, the first actuation part comprises or forms the securing element, wherein the securing element is embodied as an in particular internal screw thread engaging the main unit, in particular an end part thereof, and/or wherein the securing element can be disengaged by actuating, in particular rotating, the first actuation part.

According to another aspect of the present invention that can also be realized independently, the second actuation part can be actuated, in particular depressed, in order to at least partially (further) expand/release the drive spring / its spring force/energy and/or to move the main unit and/or the stopper from the administration position to the final position, wherein the actuation mechanism, in particular the second actuation part, comprises a blocking element/portion configured to axially block the main unit, in particular the axial movement of the main unit, in the administration position, i.e. before actuating the second actuation part, and also in the final position, i.e. after actuating the second actuation part.

With other words, the blocking element/portion stops the axial movement of the main unit and/or the stopper at the end of priming and, further, at the end of administration.

The blocking element/portion is preferably configured to axially block the main unit (only) after at least partially expanding/releasing the drive spring / its spring force/energy by means of the first actuation part and/or when the administration position is reached, in the administration state of the administration device and/or after completing priming and/or after at least partially expanding/releasing the drive spring / its spring force/energy by means of the second actuation part and/or when the final position is reached, in the final state of the administration device and/or after completing administration. However, in the initial state of the administration device and/or in the initial position of the main unit and/or the stopper, the blocking element/portion does preferably not axially block/engage the main unit.

The use of the (same) blocking element for defining the end of both priming and administration improves the dose accuracy and, thereby, minimizes the requirements of/in the manufacturing processes.

The main unit, in particular a plunger thereof, preferably comprises a first stop and a second stop, preferably wherein the first stop and the second stop are axially offset to one another and/or wherein the blocking element is adapted to axially engage the first stop in the administration position/state and/or after completing the priming process and/or wherein the (same) blocking element is adapted to axially engage the second stop in the final position/state and/or after completing administration.

According to another aspect of the present invention which can be realized also independently, the administration device comprises an indicator part indicating the state of the administration device and/or the (axial) position of the main unit and/or the stopper relative to the rack and/or the container, wherein the indicator part is rotatable, in particular rotatably driven, (directly) by the (axial) movement of the main unit, preferably only after priming and/or reaching the administration position and/or only during movement of the main unit from the administration position to the final position.

In this way, an easy and reliable (visual) indication of the state of the administration device and/or the axial position of the main unit and/or the stopper is provided, derisking misuse of the administration device due to a possible misunderstanding of the status.

The indicator part is preferably embodied as a hollow cylinder and/or a sleeve that is preferably rotabably arranged within the rack.

Preferably, the main unit axially extends through the indicator part and/or the indicator part (radially) surrounds/encases the main unit, in particular an intermediate part thereof.

The indicator part is preferably adapted to indicate the initial position, the releasing position, the administration position and/or the final position of the main unit and/or the stopper and/or the initial state, the administration state and/or the final state of the administration device, in particular by means of its rotational position relative to the main unit, the container, the stopper and/or the rack.

In order to rotate the indicator part, the main unit, in particular an intermediate part thereof, preferably comprises at least one protrusion, such as a radially orientated pin, preferably wherein the protrusion/pin protrudes into and/or engages a helical or inclined slot and/or surface of the indicator part, in particular such that an axial movement of the main unit rotates the indicator part. In this way, an easy mechanical coupling of the main unit and the indicator part is achieved.

Further, the protrusion/pin and/or an indicating means of the main unit may be visible from the outside of the administration device, thereby indicating the state of the administration device and/or the axial position of the main unit and/or the stopper, in particular the initial position, the releasing position, the administration position and/or the final position, mostly preferred additionally to the indicator part.

Mostly preferred, the protrusion/pin and/or the indicating means is adapted to indicate the state of the administration device and/or the axial position of the main unit and/or the stopper during priming and the indicator part is adapted to indicate the state of the administration device and/or the axial position of the main unit and/or the stopper during administration.

In this way, the usability is improved and/or the risk of misuse due to a possible misunderstanding of the status of the administration device is decreased.

Additionally, the indicator part is adapted to restrict the visibility into the administration device and to focus the user's / practitioner's attention to the indicator part and/or the pin, at least during priming.

A further aspect of the present invention relates to a method for operating the administration device, wherein the method comprises at least one of the following steps:
- (Manually) rotating the first actuation part, thereby axially moving the main unit, the stopper and/or the drive spring relative to the rack and/or the container, preferably together and/or as a unit, in particular by means of a leadscrew of the actuation mechanism, and/or
- (Manually) actuating, in particular rotating, the first actuation part, thereby at least partially expanding/releasing the drive spring / its spring force, in particular in the releasing position of the main unit and/or upon reaching the releasing position, and subsequently (manually) actuating, in particular depressing, the second actuation part, thereby at least partially (further) expanding/releasing the drive spring / its spring force, in particular in the administration position of the main unit, and/or
- (Manually) actuating, in particular rotating, the first actuation part, thereby (axially) moving the main unit, the stopper and/or the drive spring - in particular together and/or as one unit - from the initial position to the releasing position in which the drive spring / its spring force/energy is in particular automatically expanded/released in part, and/or
- (Manually) actuating, in particular depressing, the second actuation part, thereby at least partially expanding/releasing the drive spring / its spring force/energy which then moves, in particular pushes, the main unit and/or the stopper from the administration position to the final position, wherein the main unit is axially blocked by means of the blocking element of the actuation mechanism in both the administration position and/or before actuating the second actuation part and in the final position and/or after actuating the second actuation part, and/or
- Rotating the indicator part of the administration device by means of the (axial) movement of the main unit, in particular the pin thereof.

In this way, corresponding advantages can be achieved.

The above-mentioned aspects and features of the present invention and the aspects and features of the present invention that will become apparent from the claims and the following description can in principle be implemented independently from one another, but also in any combination or order.

Further aspects, advantages, features and properties of the present invention will become apparent from the claims and the following description of a preferred embodiment with reference to the drawings, in which:
- Fig. 1: is a schematic perspective view of a proposed administration device;
- Fig. 2: is a schematic perspective view of the administration device without casing and with a first actuation part being transparent;
- Fig. 3: is an exploded view of the administration device;
- Fig. 4: is an exploded view of the administration device, rotated by 90° about its main axis compared to Fig. 3;
- Fig. 5: is a schematic longitudinal section of the administration device in the initial position/state;
- Fig. 6: is a schematic longitudinal section of the administration device in the initial position/state, rotated by 90° about its main axis compared to Fig. 5;
- Fig. 7: is a schematic longitudinal section of the administration device in the releasing position;
- Fig. 8: is a schematic longitudinal section of the administration device in the releasing position, rotated by 90° about its main axis compared to Fig. 7;
- Fig. 9: is a schematic longitudinal section of the administration device in the administration position/state;
- Fig. 10: is a schematic longitudinal section of the administration device in the administration position/state, rotated by 90° about its main axis compared to Fig. 9;
- Fig. 11: is a schematic longitudinal section of the administration device in the final position/state;
- Fig. 12: is a schematic longitudinal section of the administration device in the final position/state, rotated by 90° about its main axis compared to Fig. 11;
- Fig. 13: is a perspective partial section of the administration device in the initial state/position;
- Fig. 14: is a perspective partial section of the administration device in the administration state/position; and
- Fig. 15: is a perspective partial section of the administration device in the final state/position.

In the figures, the same reference signs are used for the same or similar parts and components, resulting in corresponding or comparable properties, features and advantages, even if these properties, features and advantages are not repeatedly described.

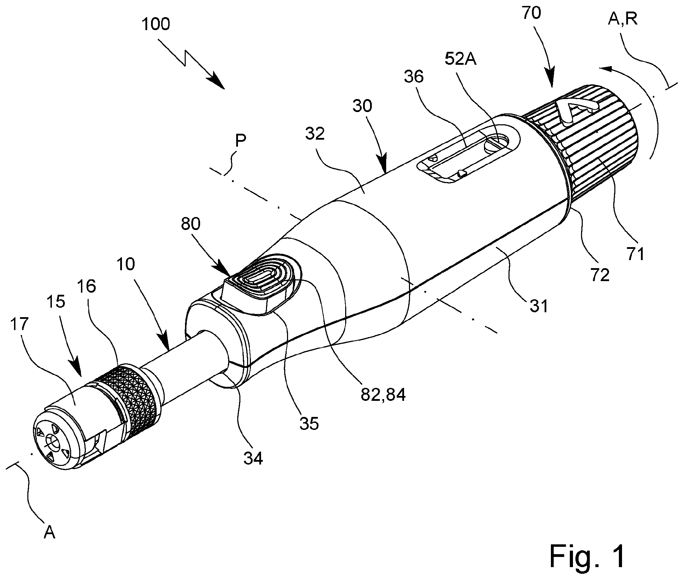

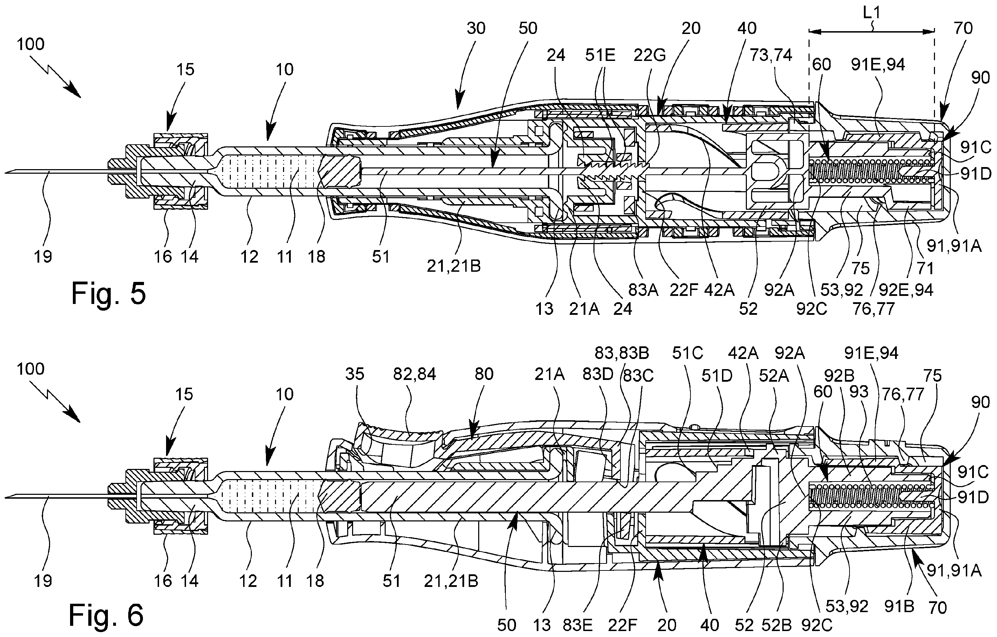

In the following, the construction and the components/parts of a proposed administration device 100 will be explained with reference to Figs. 1 to 4. The interaction of the components/parts and the operation of the administration device 100 will be explained with reference to Figs. 5 to 15, wherein - for reasons of simplicity -some reference signs have been omitted although the related features/components/parts are illustrated.

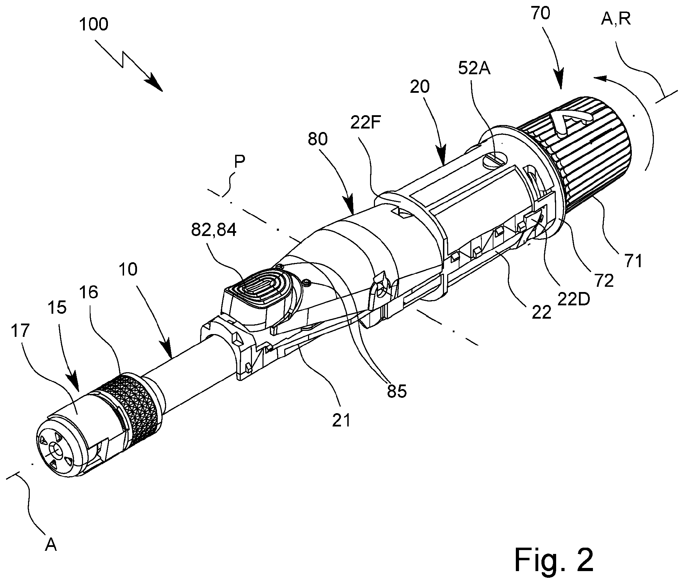

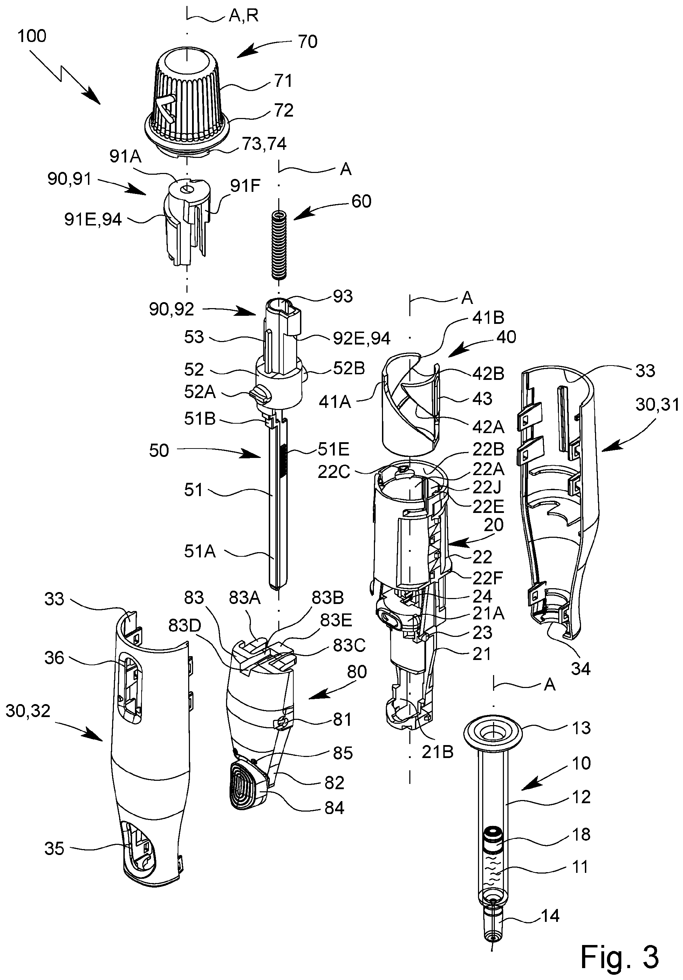

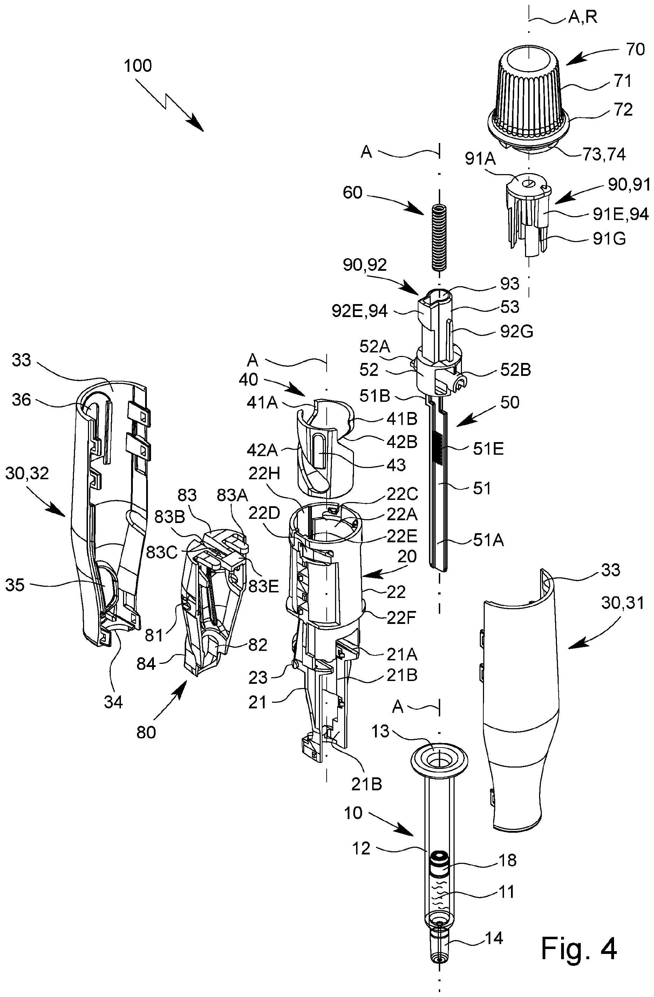

Fig. 1 is a schematic perspective view of the proposed administration device 100 in the initial state of the administration device 100. Fig. 2 corresponds to Fig. 1 with some components of the administration device 100 being masked out and shown in a transparent manner in order to illustrate the inner components of the administration device 100. Fig. 3 and Fig. 4 are exploded views of the administration device 100 from different perspectives.

The administration device 100 is of multi-part construction and/or comprises multiple parts/components, preferably wherein some or all parts of the administration device 100 are connected to each other by means of a snap/clip-on connection and/or in a screwless manner.

The terms "snap", "clip" and/or "snap/clip-on connection" is preferably understood to refer to an assembly/connection method, wherein at least two parts/components are assembled/connected to one another - in particular in a force-fitting and/or form-fitting manner - using the elasticity/flexibility of the parts/components to be assembled/connected and/or without using (additional) fastening elements, such as nails, screws, bolts or the like.

As best seen in Figs. 3 and 4, the administration device 100 preferably comprises a container 10, a rack 20, a casing 30, an indicator part 40 and an actuation mechanism having a main unit 50, a preferably compressed/pretensioned drive spring 60, a first actuation part 70, a second actuation part 80 and/or an optional primer 90.

Preferably, some or all parts/components of the administration device 100, in particular the container 10, the rack 20, the casing 30, the indicator part 40 and the actuation mechanism, the main unit 50, the first actuation part 70, the second actuation part 80 and/or the optional primer 90, are made out of, in particular injection molded from, plastics, mostly preferred polyethylene and/or polypropylene.

The administration device 100 is preferably elongated and/or rod/pen-shaped. In particular, the administration device 100 is embodied as a handheld device and/or shaped in such a manner that it ergonomically fits into a user's / practitioner's hand.

The administration device 100, in particular the container 10, the casing 30, the indicator part 40, the drive spring 60 and/or the first actuation part 70, is/are preferably at least essentially cylindrical and/or rotationally symmetric.

Preferably, the administration device 100, in particular the container 10, comprises/defines a main axis A, in particular wherein the main axis A is a central, longitudinal, symmetry and/or rotation axis of the administration device 100, in particular the container 10, and/or wherein the main axis A centrally runs through the administration device 100, in particular the container 10, the rack 20, the casing 30, the indicator part 40, the main unit 50, the drive spring 60, the first actuation part 70 and/or a needle 19 (not shown in Figs. 1 to 4) attached to the administration device 100.

In the following, spatial orientations and/or arrangements, in particular the terms "radial" or "radially" and/or "axial" or "axially", refer to the main axis A of the administration device 100, in particular the container 10, unless otherwise stated.

Preferably, the first actuation part 70 forms a (first) axial end and/or a rear end and/or the container 10, in particular its tip/outlet, forms a (second) axial end and/or a front end of the administration device 100.

In the normal position of use of the administration device 100, the container 10 preferably points away from a user/practitioner and/or towards a patient and the first actuation part 80 points towards a user/practitioner and/or away from a patient.

The casing 30 is at least essentially cylindrical and/or has the shape of a hollow cylinder, in particular having an inner and/or outer diameter that varies along the main axis A.

The casing 30 preferably encases/covers some or all parts/components of the administration device 100 radially/laterally and/or to the outside.

Preferably, the casing 30 encases/covers the container 10, the rack 20, the indicator part 40, the main unit 50, the drive spring 60, the first actuation part 70, the second actuation part 80 and/or the primer 90 at least partially or completely radially/laterally and/or to the outside.

In the present embodiment, the casing 30 partially encases/covers the container 10, the first actuation part 70 and/or the second actuation part 80 radially/laterally, whereas the casing 30 completely encases/covers the rack 20, the indicator part 40, the main unit 50, the drive spring 60 and/or the primer 90 radially/laterally and/or to the outside.

Mostly preferred, the first actuation part 70 and/or the container 10 axially protrude/protrudes out of the casing 30 and/or the second actuation part 80 radially protrude/protrudes out of the casing 30.

The casing 30 is preferably of multi-part construction. In particular, the casing 30 comprises a first casing part 31 and a second casing part 32, preferably wherein the first casing part 31 and the second casing part 32 are each embodied as a half shell and/or wherein the first casing part 31 and the second casing part 32 are connected to each other by means of a snap/clip-on connection.

The casing 30 is preferably axially open. In particular, the casing 30 comprises a first axial opening 33 and a second axial opening 34, preferably wherein the first actuation part 70 protrudes out of the first axial opening 33 and/or the container 10 protrudes out of the second axial opening 34. Mostly preferred, the first axial opening 33 is larger than the second axial opening 34.

Further, the casing 30, in particular the second casing part 32, preferably comprises a radial opening 35 and/or a casing window 36, preferably wherein the second actuation part 80 radially protrudes out of the radial opening 35 and/or wherein the interior of the administration device 100, in particular the indicator part 40, is at least partially visible through the casing window 36.

As already noted in the introductory part, the administration device 100 is adapted to administer/dispense a fluid 11, in particular a drug, to a patient, i.e. a human or animal.

Preferably, the container 10 is pre-filled with the fluid 11 and/or already contains the fluid 11 in the initial state of the administration device 100.

The administration device 100 is preferably adapted to administer/dispense the fluid 11 out of the container 10, in particular its outlet, to a patient, mostly preferred by means of its actuation mechanism, as will be explained later in greater detail.

The container 10 is preferably formed integrally / in one piece. Mostly preferred, the container 10 is made out of glass or injection molded from plastics.

The container 10 is preferably elongated and/or embodied as a hollow cylinder.

Preferably, the container 10 comprises a main body 12, preferably wherein the main body 12 is embodied as a hollow cylinder and/or wherein the inner diameter of the main body 12 is constant over the longitudinal extension of the main body 12.

The container 10 preferably comprises a first axial end 13 and a second axial end 14, in particular wherein the first axial end 13 is embodied as a flange/collar and/or comprises an enlarged outer diameter compared to the outer diameter of the main body 12 and/or the second axial end 14.

The second axial end 14 is preferably embodied as the tip of the container 10 and/or comprises or forms the outlet of the container 10.

In particular, the second axial end 14 of the container 10 is cone-shaped and/or tapered. Mostly preferred, the second axial end 14 comprises a smaller inner and/or outer diameter compared to the inner and/or outer diameter of the main body 12 and/or the first axial end 13.

Preferably, the second axial end 14 of the container 10 is embodied as a connection part for a needle 19 (not shown in Figs. 1 to 4).

As shown in Figs. 1 and 2, the administration device 100, in particular the container 10, is optionally equipped with a closure/lock 15, in particular a Luer lock, in order to protect/seal the administration device 100, in particular the container 10, mostly preferred its second axial end 14, and/or to provide a connection for the needle 19.

Preferably, the needle 19 can be attached to the container 10, in particular the second axial end 14, by means of the lock 15, mostly preferred in a form-fitting and/or force-fitting manner.

The lock 15 preferably comprises a connector 16 and/or a cap 17, preferably wherein the connector 16 and/or the cap 17 are attached to the second axial end 14 of the container 10, in particular in a form-fitting and/or force-fitting manner, and/or wherein the cap 17 axially covers the second axial end 14 and/or the connector 16, mostly preferred in the initial state of the administration device 100.

The connector 16 is preferably adapted to connect the needle 19 to the container 10, in particular its second axial end 14.

In order to connect the needle 19 to the container 10, the cap 17 can be removed from the container 10 and/or the connector 16. Subsequently, the needle 19 can be attached to the connector 16.

Preferably, the lock 15, in particular the connector 16, comprises an internal thread such that the needle 19 can be screwed into the lock 15, in particular the connector 16. In this way, a particularly firm connection of the needle 19 and lock 15 can be achieved. However, other constructional solutions are possible as well.

The administration device 100, in particular the container 10, preferably comprises a stopper 18, preferably wherein the stopper 18 is movably arranged within the container 10, in particular its main body 12.

The main body 12 of the container 10 and the stopper 18 preferably delimit a chamber for the fluid 11.

The stopper 18 is preferably made out of, in particular injection molded from, plastic material, in particular an elastomer.

The stopper 18 is preferably cylindrical and/or comprises an outer diameter that corresponds at least essentially to and/or is (slightly) larger than the inner diameter of the container 10, in particular the main body 12, preferably such that the stopper 18 presses against the inner wall of the container 10.

Preferably, the stopper 18 sealingly abuts the inner wall of the container 10, in particular the main body 12, and/or comprises or forms at least one (radial) seal, in particular such that fluid 11 cannot leak out and/or flow through a gap between the stopper 18 and the inner wall of the main body 12.

By moving/pushing the stopper 18 towards the second axial end 14 of the container 10, in particular by means of the actuation mechanism, the volume of the chamber delimited by the main body 12 and by the stopper 18 can be reduced, thereby delivering the fluid 11 contained in the container 10.

As already mentioned, the administration device 100 preferably comprises the rack 20, preferably wherein some or all parts/components of the administration device 100, are (directly) mounted on and/or - in particular either rigidly/immovably or movably - attached to and/or held by the rack 20.

Thus, the rack 20 preferably functions as a supporting/connecting/mounting/stabilizi ng structure for the other parts/components of the administration device 100, in particular the container 10, the casing 30, the indicator part 40, the main unit 50, the first actuation part 70, the second actuation part 80 and/or the primer 90.

Preferably, the container 10 and/or the casing 30 are/is rigidly/immovably held by and/or attached to the rack 20.

Preferably, the indicator part 40, the main unit 50, the first actuation part 70, the second actuation part 80 and/or the primer 90 are/is movably attached to and/or arranged within the rack 20, as will be explained later in further detail.

The rack 20 preferably comprises a - preferably radially/laterally open - mount 21 for the container 10 and/or the second actuation part 80 and a - preferably axially open - frame 22 for the indicator part 40, the main unit 50, the first actuation part 70 and/or the primer 90.

The mount 21 and the frame 22 are preferably made out of the same material and/or formed, in particular injection molded, in one piece.

The rack 20, in particular the mount 21, is preferably adapted to immovably hold the container 10, preferably in a clamped manner and/or in particular in such a way that the container 10 is radially and axially held within and/or by means of the rack 20, in particular the mount 21.

The container 10 is preferably radially/laterally inserted, in particular pressed/clipped, into the rack 20, mostly preferred the mount 21.

To this end, the inner shape/contour of the mount 21 preferably corresponds at least essentially to the outer shape/contour of the container 10.

The mount 21 preferably comprises an in particular slot-shaped socket 21A for the first axial end 13 of the container 10 and/or a preferably half-shell-like retainer 21B for the main body 12 of the container 10.

Mostly preferred, the first axial end 13 of the container 10 is (radially) inserted, in particular pressed/clipped, into the socket 21A and/or the main body 12 is (radially) inserted, in particular pressed/clipped, into the retainer 21B, in particular such that the container 10 is immovably held by the rack 20, in particular the mount 21.

The rack 20, in particular the frame 22, is preferably adapted to (axially) receive and/or (radially) support the indicator part 40, main unit 50, the first actuation part 70, the second actuation part 80 and/or the primer 90, in particular such that the indicator part 40 and/or the second actuation part 70 can be rotated relative to the rack 20 and/or are axially held by the rack 20, in particular the frame 22, and/or such that the main unit 50 and/or the primer 90 can axially move relative to and/or within the rack 20, in particular the frame 22, as will be explained in the following.

The indicator part 40, the main unit 50, the first actuation part 70 and/or the primer 90 are/is preferably axially inserted into the rack 20, mostly preferred the frame 22. Preferably, the indicator part 40, the main unit 50 and/or the primer 90 are/is completely inserted and/or the first actuation part 70 is partially inserted into the rack 20, mostly preferred the frame 22.

The rack 20, in particular the frame 22, preferably comprises an in particular at least essentially cylindrical receptacle 22A for the indicator part 40, the main unit 50, the first actuation part 70 and/or the primer 90, preferably wherein the receptacle 22A is axially open.

Mostly preferred, the rack 20, in particular the frame 22, comprises an axial opening 22B for insertion of the indicator part 40, the main unit 50, the first actuation part 70 and/or the primer 90 into the rack 20.

As already mentioned, the first actuation part 70 is preferably cap-like and/or at least essentially cylindrical.

The first actuation part 70 preferably comprises/forms an axial end of the administration device 100.

The first actuation part 70 preferably comprises a handle/dial 71, a collar 72 and/or a connector 73, preferably wherein the collar 72 is (axially) arranged between the handle/dial 71 and the connector 73 and/or wherein the outer diameter of the collar 72 is larger than the outer diameter of the handle/dial 71 and/or the connector 73.

The handle 71 preferably protrudes out of the rack 20, in particular its axial opening 22B. The collar 72 preferably covers the rack 20 and/or the casing 30 axially, in particular the gap between the connector 73 and the rack 20 and/or the gap between the rack 20 and the casing 30.

The first actuation part 70 is preferably directly attached, in particular axially secured, to the rack 20, in particular the frame 22.

Preferably, the first actuation part 70, in particular its connector 73, is axially attached, in particular pressed/clipped into or onto, the rack 20, in particular the frame 22, mostly preferred into the axial opening 22B.

Preferably, the rack 20, in particular the frame 22, comprises a plurality of retaining elements 22C that radially engage the first actuation part 70, preferably the connector 73 and/or corresponding protrusions and/or recesses formed therein (not shown), mostly preferred such that the first actuation part 70 is axially secured to the rack 20, in particular the frame 22.

However, it is preferred that the first actuation part 70 can be moved, in particular rotated, relative to the rack 20, preferably in a predefined direction of rotation, as indicated in Fig. 1 and 2 by an arrow.

Preferably, the actuation mechanism, in particular the first actuation part 70, can be actuated by manually rotating the first actuation part 70 relative to the rack 20.

In order to facilitate rotation of the first actuation part 70, the outer surface of the first actuation part 70, in particular the handle 71, might be at least partially grooved/ripped or otherwise profiled.

The administration device 100, in particular the actuation mechanism and/or the first actuation part 70, preferably comprises/defines a rotation axis R, preferably wherein the first actuation part 70 is rotatable about the rotation axis R.

The rotation axis R preferably corresponds to/coincides with the main axis A of the administration device 100. However, other solutions are possible as well, wherein the rotation axis R differs from the main axis A, in particular wherein the rotation axis R is inclined relative to the main axis A.

It is preferred that the first actuation part 70 can be rotated only in one direction and/or in that the administration device 100, in particular the rack 20, comprises a back-rotation lock, in particular a ratchet, for the first actuation part 70.

The intended direction of rotation might be indicated on the first actuation part 70, in particular its handle 71, by means of a label, a mark or the like, e.g. as shown in Figs. 1 to 3.

In the present embodiment and as best seen in Fig. 4, the rack 20, in particular the frame 22, comprises at least one back-rotation stop 22D and the first actuation part 70 comprises at least one (corresponding) back-rotation stop 74, preferably wherein the back-rotation stop 22D of the rack 20 and the back-rotation stop 74 of the first actuation part 70 radially interact with one another, in particular in such a manner that only a rotation of the actuation part 70 in the intended direction of rotation is possible and/or such that a back-rotation of the first actuation part 70, i.e. in a direction opposite to the intended direction of rotation, is prevented or limited.

In particular, the back-rotation stop 74 of the first actuation part 70 and the back-rotation stop 22D of the rack 20 engage with one another in a form-fitting manner in a direction opposite to the intended direction of rotation and/or glide past each other, e.g. by elastically/temporarily deforming/bending the back-rotation stop 74 of the first actuation part 70 and/or the back-rotation stop 22D of the rack 20, in the intended direction of rotation.

In the present embodiment, the rack 20, in particular the frame 22, comprises one back-rotation stop 22D in the form of a flexible arm extending into the interior of the rack 20, in particular the receptacle 22A, and the first actuation part 70 comprises a plurality of back-rotation stops 74 in the form of saw teeth. However, other constructional solutions are possible as well.

The back-rotation lock de-risks misuse of the administration device 100 by reversal of the first actuation part 70 and might provide a haptic/audible feedback during priming, thereby increasing the user-friendliness of the administration device 100.

Optionally, the rack 20 and/or the first actuation part 70 comprise/comprises an actuation stop 22E in order to limit the actuation, in particular rotation, of the first actuation part 70 relative to the rack 20, in particular in the rotation direction.

As best seen in Fig. 4, in the present embodiment the actuation stop 22E is embodied as a protrusion extending radially into the axial opening 22B / the receptacle 22A of the rack 20.

The first actuation part 70 preferably comprises a corresponding actuation stop (not shown), preferably wherein the actuation stop of the first actuation part 70 abuts the actuation stop 22E of the rack 20 in circumferential direction when a predefined rotary orientation of the first actuation part 70 is reached, in particular when reaching the releasing position.

As already mentioned, the administration device 100 comprises - in addition to the first actuation part 70 - a further, second actuation part 80.

The second actuation part 80 is preferably radially/laterally attached to the rack 20, in particular the mount 21.

Mostly preferred, the second actuation part 80 and the container 10 are mounted to the rack 20, in particular the mount 21, from opposing sides. In this way, the second actuation part 80 and the container 10 can be mounted independent from one another to the rack 20.

Preferably, second actuation part 80 is preferably movably, in particular pivotably, attached to the rack 20, in particular the mount 21.

The second actuation part 80 is preferably of shell-like construction and/or covers the rack 20, in particular the mount 21, laterally, to one side and/or partially.

Preferably, the second actuation part 80 is clipped into or onto the rack 20, in particular the mount 21, mostly preferred in a pivotable manner.

The administration device 100, in particular the rack 20, mostly preferred the mount 21, and/or the second actuation part 80, define/defines a pivot axis P, preferably wherein the second actuation part 80 can be pivoted relative to the rack 20 about the pivot axis P.

As best seen in Figs. 1 and 2, the pivot axis P of the second actuation part 80 is preferably arranged transversely, in particular at least essentially perpendicular, to the main axis A of the administration device 100 and/or the rotation axis R of the first actuation part 70.

Preferably, the rack 20 comprises or forms a pair of pivot pins 23, preferably wherein the pivot pins 23 define the pivot axis P.

The pivot pins 23 are preferably embodied as protrusions/pins that extend laterally and/or in radial direction from the rack 20.

The second actuation part 80 preferably comprises a pair of sockets 81, preferably wherein each socket 81 is adapted to receive a corresponding pivot pin 23 and/or wherein each pivot pin 23 is (directly) clipped into one corresponding socket 81 of the second actuation part 80.

However, other constructional solutions are also possible, in particular wherein the second actuation part 80 comprises or forms the pivot pins 23 and the rack 20 comprises or forms the sockets 81 for the pivot pins 23.

The second actuation part 80 is preferably elongated and/or comprises a first (axial) end 82 and a second (axial) end 83, preferably wherein the pivot axis P extends through the second actuation part 80 at least essentially centrally and/or between the first end 82 and the second end 83.

Thus, it is preferred that the second actuation part 80 is tiltable relative to the rack 20 and/or that the second actuation part 80 is embodied as a toggle.

Preferably, the second actuation part 80, in particular the first end 82, comprises or forms a preferably oval shaped button 84, in particular wherein the button 84 is embodied as a radial protrusion and/or projects radially/laterally out of the administration device 100, in particular through the radial opening 35 of the casing 30, in particular the second casing part 32.

Preferably, the second actuation part 80 is actuatable by depressing the button 84, in particular towards the rack 20 and/or the main axis A.

By actuating the second actuation part 80, in particular by depressing the button 84, the second end 83 of the second actuation part 80 is preferably raised from and/or moved out of the rack 20 and/or towards to casing 30.

The second end 83 of the second actuation part 80 is preferably embodied as a radial, in particular plate-shaped, protrusion and/or projects radially into the rack 20, in particular the frame 22 of the rack 20, and/or towards the main axis A.

Preferably, the second actuation part 80, in particular the second end 83 thereof, comprises or forms at least one actuation spring 83A, mostly preferred in form of a flexible protrusion/arm.

The rack 20, preferably the frame 22, in particular the receptacle 22A, preferably comprises a bottom 22F, preferably wherein the second end 83 of the second actuation part 80, in particular the actuation spring 83A, axially rests against the bottom 22F. With other words, the bottom 22F preferably functions as a counter bearing and/or forms a bearing surface for the actuation spring 83A.

The actuation spring 83A is preferably pretensioned, in particular against the bottom 22F, in the initial state of the administration device 100, mostly preferred such that the second actuation part 80 is actuatable against the spring force/energy of the actuation spring 83A. In this way, the risk of an unintended actuation of the second actuation part 80 is reduced.

The second actuation part 80, in particular the second end 83 thereof, preferably comprises or forms a frame 83B and/or an aperture 83C, preferably wherein the main unit 50 extends through the frame 83B and/or the aperture 83C.

By actuating the second actuation part 80, in particular the button 84, the (radial) position of the frame 83B and/or the aperture 83C relative to the main unit 50 is changed, thereby axially blocking or releasing the main unit 50, as will be explained later in further detail.

Usually, medical devices, like the administration device 100, are sterilized, in particular by means of and/or exposure to a sterilant, in particular a sterilizing gas, such as ethylene oxide. In such sterilizing processes it is of particular importance to expose all surfaces of the medical devices to the sterilant a user/practitioner might get into contact with.

To this end, the casing 30 and/or the second actuation part 80 preferably comprise/comprises at least one spacer 85, preferably wherein the spacer 85 is adapted to maintain a gap between the casing 30 and the second actuation part 80 in particular in close proximity to the button 84 and/or even in the initial/unactuated state of the administration device 100.

In this way, the contact surface is maximized during sterilization of the administration device 100. In particular, a sterilant, such as ethylene oxide, can flow into the gap between the casing 30 and the second actuation part 80 during the sterilization process such that the entire button 84 is exposed to the sterilant during sterilization.

As already mentioned, the administration device 100, in particular the actuation mechanism thereof, comprises the main unit 50, preferably wherein the main unit 50 is movably arranged within and/or relative to the rack 20.

The main unit 50 is preferably adapted to (directly) act on and/or abut the stopper 18. In particular, the main unit 50 is adapted to push the stopper 18 towards the outlet and/or fluid 11 of the container 10.

However, it is preferred that the stopper 18 is not rigidly attached to the main unit 50, in particular its tip. Preferably, the stopper 18 merely rests against and/or is loosely connected to the main unit 50, in particular its tip. With other words, the main unit 50 and the stopper 18 are configured to connect merely in compression and/or when the main unit 50 is pushed against the stopper 18. The main unit 50 and the stopper 18 are preferably configured to decouple in tension and/or when the main unit 50 is moved/pulled out of the container 10. This prevents drawing air into the container 10, for example during the sterilizing process.

In the initial state of the administration device 100, the stopper 18 might also be axially spaced apart from the main unit 50 such that the main unit 50 axially abuts the stopper 18 only upon actuation of the first actuation part 70.

The main unit 50 is preferably elongated and/or rod-shaped.

Mostly preferred, the main unit 50 extends (completely) axially through the administration device 100, preferably the rack 20, in particular from the first actuation part 70 through the rack 20 into the container 10.

In particular, the main unit 50 extends out of the casing 30, in particular the second axial opening 34.

The main unit 50 preferably comprises a plunger/connecting rod 51, an intermediate part 52 and/or an end part 53, preferably wherein the intermediate part 52 is (axially) arranged between the plunger/connecting rod 51 and the end part 53.

The plunger/connecting rod 51, hereinafter referred to as plunger rod 51, preferably comprises or forms a (first) axial end and/or the tip of the main unit 50. Preferably, the plunger rod 51 extends into the container 10 and/or (directly) faces the stopper 18.

The end part 53 of the main unit 50 preferably comprises or forms a (second) axial end of the main unit 50. Preferably, the end part 53 faces away from the stopper 18 and/or mechanically interacts with and/or at least partially extends into the first actuation part 70, as will be explained later with greater detail.

The plunger rod 51 is preferably elongated and/or rod-shaped.

In the present embodiment, the plunger rod 51 preferably comprises an at least essentially rectangular cross-section. However, other constructional solutions are possible as well, in particular wherein the plunger rod 51 comprises a circular cross-section.

The outer dimensions of the plunger rod 51 are preferably smaller than the inner diameter of the container 10, in particular its main body 12, preferably such that the plunger rod 51 can move freely and/or without friction within the container 10.

The plunger rod 51 preferably comprises a front portion 51A and a rear portion 51B.

The front portion 51A is preferably the portion of the plunger rod 51 that at least partially extends into the container 10 and/or (directly) faces/abuts the stopper 18.

The rear portion 51B of the plunger rod 51 is preferably the portion of the plunger rod 51 that projects out of the container 10 and/or comprises or forms the transition between the front portion 51A and the intermediate part 52 of the main unit 50.

The shape of the plunger rod 51, in particular its front portion 51A, is preferably uniform. However, the shape of the plunger rod 51, in particular its rear portion 51B and/or the portion adjacent to the intermediate part 52, might also change in the direction of the main axis A.

Preferably, the rear portion 51B of the plunger rod 51 is stepped and/or radially shifted compared to the front portion 51A of the plunger rod 51.

Preferably, the plunger rod 51, in particular its rear portion 51B, comprises or forms a first stop 51C and/or a second stop 51D, preferably wherein the first stop 51C and/or the second stop 51D are/is formed by a (radial) step/protrusion of the plunger rod 51, in particular its rear portion 51B, best seen in Fig. 6.

The second actuation part 80, in particular the frame 83B, preferably comprises or forms a blocking element/portion 83D, preferably wherein the blocking element/portion 83D is configured to axially block the main unit 50, in particular by axially engaging the first stop 51C and/or the second stop 51D.

By actuation of the first actuation part 70 and/or the second actuation part 80, the main unit 50 is preferably moved towards the outlet of the container 10 and/or further into the container 10 and/or away from the first actuation part 70.

Preferably, the direction of movement of the main unit 50 is parallel to, in particular along, the main axis A.

In addition or as an alternative to the back-rotation lock for the first actuation part 70, the administration device 100 might be equipped with a return lock, in particular a ratchet, for the main unit 50, preferably in order to prevent or at least limit a back-movement of the main unit 50, in particular (only) during priming and/or during the sterilization process of the administration device 100.

Preferably, the rack 20 and the main unit 50 mechanically and/or axially interact with one another in such a manner that the main unit 50 can only be moved in the (intended) direction of movement and/or further into the container 10 and/or further away from the first actuation part 70 and/or that a back-movement of the main unit 50, i.e. a movement of the main unit 50 out of the container 10 and/or away from the container outlet, is prevented or at least limited, in particular (only) during priming.

As best seen in Fig. 5 that shows a longitudinal section of the administration device 100 in the initial position/state, the rack 20, in particular the bottom 22F of the receptacle 22A, preferably comprises an opening/slot 22G for the main unit 50, in particular the plunger rod 51, preferably wherein the main unit 50, in particular the plunger rod 51, extends through the opening/slot 22G of the rack 20.

The opening 22G preferably guides the main unit 50 radially and/or prevents or limits a potential rotation of the main unit 50 relative to the rack 20.

The rack 20, in particular the frame 22, preferably comprises at least one return stop 24, best seen in Fig. 5.

The main unit 50, in particular the plunger rod 51, preferably comprises a (corresponding) return stop 51E, preferably wherein the return stop 24 of the rack 20 and the return stop 51E of the main unit 50 form the return lock of the administration device 100 and/or axially interact with one another in such a way that the main unit 50 can only be moved into the (intended) direction of movement and/or towards and/or further into the container 10 and/or that a movement of the main unit 50 in a direction opposite to the (intended) direction of movement and/or out of the container 20 and/or towards the first actuation part 70 is prevented or at least limited, in particular only or at least during priming and/or actuating the first actuation part 70.

In particular, the return stop 24 of the rack 20 and the return stop 51E of the main unit 50 engage with one another in a form-fitting manner in a direction opposite to the intended direction of movement and/or glide past each other, e.g. by elastically/temporarily deforming/bending the return stop 24 of the rack 20 and/or the return stop 51E of the main unit 50, in the intended direction of movement.

In the present embodiment, the main unit 50, in particular the plunger rod 51, preferably comprises a plurality of return stops 51E, in particular in form of saw teeth, arranged on opposite sides of the plunger rod 51, in particular its front portion 51A.

The rack 20 preferably comprises a plurality of corresponding return stops 24 in form of (two) flexible arms that radially engage the corresponding return stops 51E of the main unit 50 on both sides.

Mostly preferred, the return stops 51E arranged on one side of the plunger rod 51 are axially offset to the return stops 51E arranged on the other side of the plunger rod 51, in particular such that the peak of the return stops 51E on either side of the plunger rod 51 do not align. In this way, the axial position of the main unit 50 relative to the rack 20 is secured in small steps.

Preferably, the return stops 24 of the rack 20 and the return stops 51E of the main unit 50 form the return lock, in particular ratchet.

The plurality of return stops 51E is preferably arranged only in a portion of the main unit 50, in particular the plunger rod 51, mostly preferred its front portion 51A, and/or not over the entire main unit 50, in particular not over the entire plunger rod 51, mostly preferred not over its entire front portion 51A.

As already mentioned, it is preferred that the back-movement of the main unit 50, i.e. a movement of the main unit 50 out of the container 10, is prevented or at least limited only or at least during priming.

With other words, the plurality of return stops 51E of the main unit 50 and the return stops 24 of the rack 20 preferably only or at least mechanically interact with one another and/or (axially) engage one another during priming and/or actuation of the first actuation part 70.

The return lock might provide an audible/haptic feedback during priming, in particular in addition to and/or distinguishable from the audible/haptic feedback of the back-rotation lock, mostly preferred such that the user/practitioner can clearly discern priming and/or when the administration position/state is reached.

Referring again to Figs. 3 and 4, the main unit 50, in particular the intermediate part 52, preferably comprises or forms at least one an indicating means. In the present embodiment, the indicating means is embodied as pin 52A/52B, preferably wherein the pin 52A/52B protrudes from the main unit 50 radially and/or towards and/or into the rack 20, in particular the frame 22. However, other constructional solutions are possible as well, e.g. wherein the indicating means is embodied as a preferably arc-shaped bar, in particular protruding from the main unit 50 radially.

The rack 20 preferably comprises an in particular longitudinal and/or axial recess 22H/22J, preferably wherein the pin 52A/52B is guided within the recess 22H/22J, in particular in the circumferential direction and/or such that a rotation of the main unit 50 relative to the rack 20 is prevented or at least limited.

Preferably, the indicating means, in particular the pin 52A/52B, is visible from the outside of the administration device 100 and/or through the casing window 36, as shown in Fig. 1. In this way, the axial position of the main unit 50 relative to the rack 20 is indicated and/or visible to a user/practitioner of the administration device 100. Thus, it is preferred to use the indicating means, in particular the pin 52A/52B, for both guidance of the main unit 50 and indication of the axial position of the main unit 50.

The rack 20 is preferably made at least partially out of a transparent material. Mostly preferred, at least the portion of the rack 20 covering the indicating means, in particular the pin 52A/52B, is at least partially transparent, such that the indicating means, in particular the pin 52A/52B, is visible from the outside and/or through the casing window 36. However, the rack 20 might also be equipped with a cutout such that the indicating means, in particular the pin 52A/52B, is visible from the outside and/or through the casing window 36.

In the present embodiment, the main unit 50 preferably comprises a first pin 52A and a second pin 52B and the rack 20 preferably comprises a first recess 22H and a second recess 22J, in particular on opposite sides of the main unit 50 and rack 20, preferably wherein the first pin 52A extends into the first recess 22H and the second pin 52B extends into the second recess 22J. In this way, the main unit 50 is guided on both sides and the risk of jamming is reduced.

As already mentioned, the administration device 100 preferably comprises an indicator part 40 to indicate the state of the administration device 100 and/or the axial position of the main unit 50 relative to the rack 20.

The indicator part 40 is preferably embodied as a hollow cylinder and/or a sleeve.

The indicator part 40 is preferably arranged within the rack 20, in particular the receptacle 22A.

The main unit 50, in particular the intermediate part 52, is preferably arranged within the indicator part 40.

Preferably, the indicator part 40 radially encases the main unit 50, in particular the intermediate part 52, and/or is arranged radially between the main unit 50, in particular the intermediate part 52, and the rack 20, in particular the frame 22.

However, the main unit 50, in particular its indicating means, might also cover the indicator part 40 partially and/or to the outside, in particular when being embodied as a preferably arc-shaped bar.

The indicator part 40 is preferably rotatable relative to and/or within the rack 20, in particular the receptacle 22A, and/or relative to and/or around the main unit 50, in particular the intermediate part 52. However, it is preferred that the indicator part 40 is axially secured within the rack 20 and/or by means of the rack 20.

According to one aspect of the present invention, the indicator part 40 is preferably rotatable (directly) by means of the axial movement of the main unit 50, in particular the pin 52A/52B, thereof. Preferably, the indicator part 40 is rotatably driven (directly) by the (axial) movement of the main unit 50, in particular the pin 52A/52B thereof.

With other words, actuating, in particular rotating, the first actuation part 70 preferably causes an axial movement of the main unit 50 which, in turn, causes a rotation of the indicator part 40.

According to a preferred embodiment (not shown), the indicator part 40 is rotatably driven (directly) by the (axial) movement of the main unit 50 only after reaching the administration position/state and/or during the movement from the administration position/state to the final position/state, in particular in order to indicate when the final position/state is reached. In this embodiment, the indicator part 40 preferably does not rotate during priming.

The indicator part 40 preferably comprises an axial opening 41A/41B and/or a helical/inclinded slot/surface 42A/42B, preferably wherein the pin 52A/52B can axially be inserted into the opening 41A/41B and/or the helical/inclinded slot/surface 42A/42B of the indicator part 40, in particular through the opening 41A/41B into the helical slot/surface 42A/42B, and/or wherein the main unit 50, in particular the pin 52A/52B, axially abuts/engages the helical/inclinded slot/surface 42A/42B, in particular only after reaching the administration position/state and/or during the movement from the administration position/state to the final position/state.

Preferably, the opening 41A/41B continues into the helical/inclinded slot/surface 42A/42B and/or the opening 41A/41B is formed by an (axial) end of the helical/inclinded slot/surface 42A/42B and/or the helical/inclinded slot/surface 42A/42B helically extends in the circumferential direction of the indicator part 40.

The pin 52A/52B preferably radially protrudes into the slot/surface 42A/42B such that an axial movement of the main unit 50, in particular the pin 52A/52B, rotates the indicator part 40 relative to the rack 20 and/or the main unit 50.

In the present embodiment, the indicator part 40 comprises a first axial opening 41A and/or a first helical slot/surface 42A for the first pin 52A and a second axial opening 41B and/or a second helical slot/surface 52B for the second pin 52B. In this way, jamming of the indicator part 40 can be prevented.

According to a preferred embodiment (not shown), the main unit 50 axially engages/abuts the helical slot/surface 42A/42B only after reaching the administration position/state and/or during the movement from the administration position/state to the final position/state, in particular such that the indicator part 40 is rotatably driven by the (axial) movement of the main unit 50 only after reaching the administration position/state and/or during the movement from the administration position/state to the final position/state.

The indicator part 40 is preferably adapted to indicate the state of the administration device 100 and/or the axial position of the main unit 50. To this end, the indicator part 40 preferably comprises at least one indicating means 43, such as a label, a mark or the like, on the outer surface of the indicator part 40, as best seen in Fig. 4.

The indicator part 40, in particular the indicating means 43, is preferably visible from the outside and/or through the casing window 36, in particular permanentely, during priming, during administration and/or in the initial position/state, the administration position/state and/or the final position/state.

As already mentioned, the administration device 100 can be primed, in particular by actuating, mostly preferred rotating, the first actuation part 70.

By actuating, in particular rotating, the first actuation part 70, the main unit 50 is (axially) moved, in particular such that the plunger rod 51 moves/pushes the stopper 18 further into the container 10 and/or towards the outlet thereof.

The administration device 100, in particular the actuation mechanism, preferably comprises the primer 90 that mechanically connects the main unit 50 with the first actuation part 70 and/or mechanically interacts with the first actuation part 70, in particular such that an actuation, in particular rotation, of the first actuation part 70 causes an axial movement of the main unit 50, in particular for priming of the administration device 100.

In the following, the construction and features of the primer 90 will be described with reference to Figs. 5 and 6, wherein Fig. 5 is a schematic longitudinal section of the administration device 100 in the initial position/state and Fig. 6 is a schematic longitudinal section of the administration device 100 in the initial position/state, rotated by 90° about its main axis A compared to Fig. 5.

As already mentioned, the primer 90 is preferably at least partially arranged within the first actuation part 70, at least in the initial state/position.

The first actuation part 70 preferably comprises or forms a receptacle 75, preferably wherein the primer 90 is arranged within and/or extends into the receptacle 75 of the first actuation part 70, at least in the initial state/position.

However, the first actuation part 70, in particular its connector 73, might also be inserted and/or at least partially arranged within the primer 90.

The primer 90 is preferably of multi-part construction and/or comprises multiple parts/components.

The primer 90 preferably comprises a first primer part 91 and a second primer part 92.

The second primer part 92 is preferably rigidly connected to or formed by the main unit 50, in particular its end part 53. Mostly preferred, the second primer part 92 and the main unit 50 are formed in one piece / integrally. However, the second primer part 92 might also be a separate part/component.

The primer 90, in particular the first primer part 91 and/or the second primer part 92, is/are preferably embodied as a hollow cylinder.

Preferably, the primer 90, in particular the first primer part 91 and/or the second primer part 92, comprises/comprise and/or delimits/delimt a spring chamber 93, in particular radially and axially.

The drive spring 60 is preferably arranged within the primer 90, in particular the spring chamber 93, and/or encapsulated by the primer 90, in particular the first primer part 91 and the second primer part 92.

The first primer part 91 and the second primer part 92 are preferably axially releasable connected to one another. In particular, the first primer part 91 and the second primer part 92 are (axially) fitted/plugged into each other.

In particular, the drive spring 60 / its spring force/energy is adapted to move, in particular push, the first primer part 91 and the second primer part 92 apart from each other.

Preferably, the drive spring 60 is pretensioned, mostly preferred compressed, in the initial state of the administration device 100, in particular such that the first primer part 91 and the second primer part 92 are pushed apart from one another by means of the drive spring 60.

Preferably, the drive spring 60 is embodied as coil/helical spring and/or made out of metal, in particular stainless steel.

The first primer part 91 and the second primer part 92 preferably each comprise a bottom 91A/92A and/or a preferably cylindrical wall 91B/92B, in particular wherein the bottom 91A of the first primer part 91 and the bottom 92A of the second primer part 92 axially delimit the spring chamber 93 and/or the wall 91B of the first primer part 91 and/or the wall 92B of the second primer part 92 radially delimit/delimits the spring chamber 93.

Preferably, the bottom 91A of the first primer part 91 and the bottom 92A of the second primer part 92 are arranged on (axially) opposed sides and/or delimit the spring chamber 93 on (axially) opposed sides.

In the present embodiment, the second primer part 92 is partially inserted into the first primer part 91, in particular such that the wall 91B of the first primer part 91 at least partially encases the wall 92B of the second primer part 92 and/or is arranged (directly) adjacent to the first actuation part 70.

However, the first primer part 91 might also be inserted into the second primer part 92.

The drive spring 60 preferably axially rests on and/or presses against the bottom 91A of the first primer part 91 and the bottom 92A of the second primer part 92.

The first primer part 91, in particular its bottom 91A, preferably comprises or forms a (first) spring seat 91C and the second primer part 92, in particular its bottom 92A, preferably comprises or forms a (second) spring seat 92C for the drive spring 60, in particular for the respective (axial) ends of the device spring 60.

In particular, in order to avoid kinking of the drive spring 60, the bottom 91A of the first primer part 91 and/or the bottom 92A of the second primer part 92 may be equipped with and/or form a spring pin 91D that extends axially into the drive spring 60.

According to another aspect of the present invention, the administration device 100, in particular its actuation mechanism, preferably comprises a leadscrew, wherein the first actuation part 70, on the one hand, and the main unit 50 and/or the primer 90, on the other hand, (directly) movably interact with one another and/or are (directly) movably connected to another by means of the leadscrew and/or wherein the first actuation part 70 is (manually) rotatable, in particular relative to the rack 20 and/or the container 10, in order to axially move the main unit 50, the drive spring 60 and/or the primer 90 in particular relative to the rack 20 and/or the container 10, mostly preferred such that the rotation of the first actuation part 70 moves the main unit 50, the drive spring 60 and/or the primer 90 out of the initial position into the releasing position and/or towards the administration position, axially and/or towards the stopper 18 and/or the outlet of the container 10.

Thus, the leadscrew is adapted to translate the (manual) rotation of the first actuation part 70 into a linear, in particular axial, motion of the main unit 50, the drive spring 60 and/or the primer 90.

The first actuation part 70 preferably comprises a screw thread 76 and/or the primer 90 preferably comprises a screw thread 94, preferably wherein the screw thread 76 of the first actuation part 70 and the screw thread 94 of the primer 90 (mechanically) interact with one another.

In the present embodiment, the screw thread 76 of the first actuation part 70 is embodied as an internal screw thread and the screw thread 94 of the primer 90 is embodied as an external screw thread. However, the screw thread 76 of the first actuation part 70 might also be embodied as an external screw thread and the screw thread 94 of the primer 90 might also be embodied as an internal screw thread.

Thus, it is preferred that the primer 90 is screwed into the first actuation part 70, in particular its receptacle 75, at least in the initial state/position, and/or can be screwed out of the first actuation part 70, in particular its receptacle 75, by rotating the first actuation part 70.

The screw thread 76 of the first actuation part 70 is preferably formed by a helical protrusion that extends into the receptacle 75.

The screw thread 94 of the primer 90 is preferably formed by a ridge and/or a recess on the wall 91B of the first primer part 91 and/or on the wall 92B of the second primer part 92.

Mostly preferred, the screw thread 94 is formed by both the first primer part 91, in particular its wall 91B, and the second primer part 92, in particular its wall 92B.

The first primer part 91, in particular its wall 91B, preferably comprises or forms a (first) thread part 91E and the second primer part 92, in particular its wall 92B, preferably comprises or forms a (second) thread part 92E, in particular wherein the thread part 91E and the thread part 92E (together) form the - in particular continuous - screw thread 94 of the primer 90.

As best seen in Fig. 3 and 4, the thread part 91E and/or the wall 91B of the first primer part 91 is preferably partially recessed / cut out.

The first primer part 91, in particular its wall 91B, preferably comprises a slot 91F for the second primer part 92, in particular its thread part 92E.

Preferably, the thread part 92E of the second primer part 92 can be (axially) or is (axially) inserted into the first primer part 91, in particular its slot 91F, such that the thread part 91E of the first primer part 91 and the thread part 92E of the second primer part 92 form the (continuous) screw thread 94 of the primer 90.

The thread part 92E of the second primer part 92 is preferably formed by a radial protrusion of the wall 92B of the second primer part 92 that protrudes through/into the wall 91B of the first primer part 91, in particular the slot 91F, and/or that sits flush with the outer surface of the wall 92B of the second primer part 92.

With other words, the second primer part 92, in particular its thread part 92E, engages the first primer part 91, in particular its wall 91B and/or the slot 91F, radially/rotationally and/or in a circumferential direction and/or such that the first primer part 91 and the second primer part 92 cannot rotate relative to one another, preferably at least during priming and/or until reaching the administration position.

Optionally, the first primer part 91 and the second primer part 92 might engage one another with additional elements. In the present embodiment, the first primer part 91, in particular its wall 91B, comprises a groove 91G and the second primer part 92, in particular its wall 92B, comprises a corresponding ridge 92G, preferably wherein the ridge 92G engages the groove 91G, radially/rotationally and/or in a circumferential direction, preferably at least during priming and/or until reaching the administration position.

In the following, the different states of the administration device 100 and the interaction between the parts/components of the administration device 100 during use will be explained with reference to the Figs. 5 to 15.

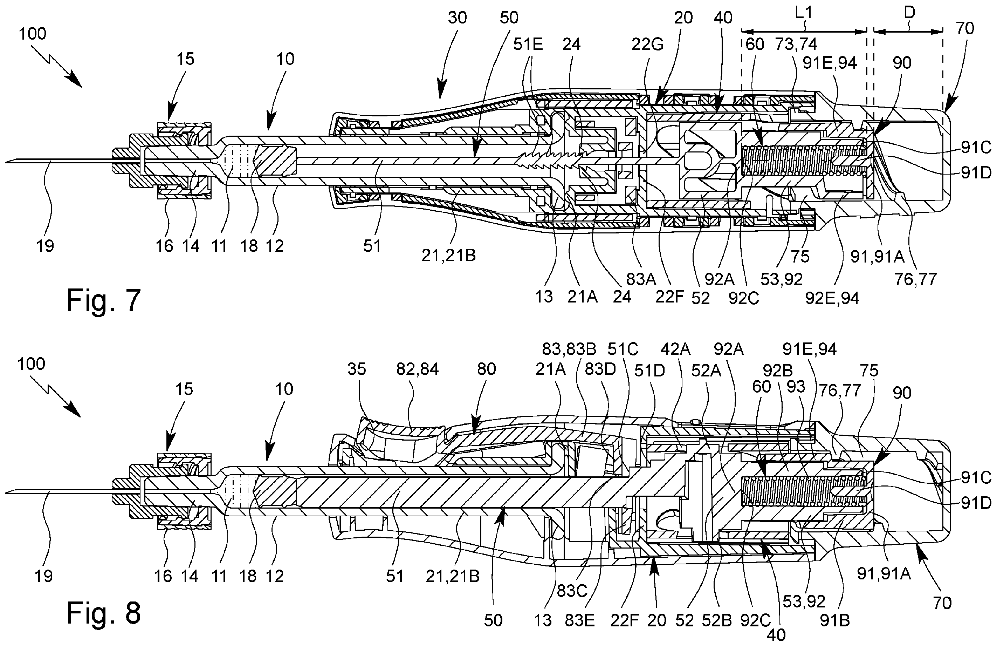

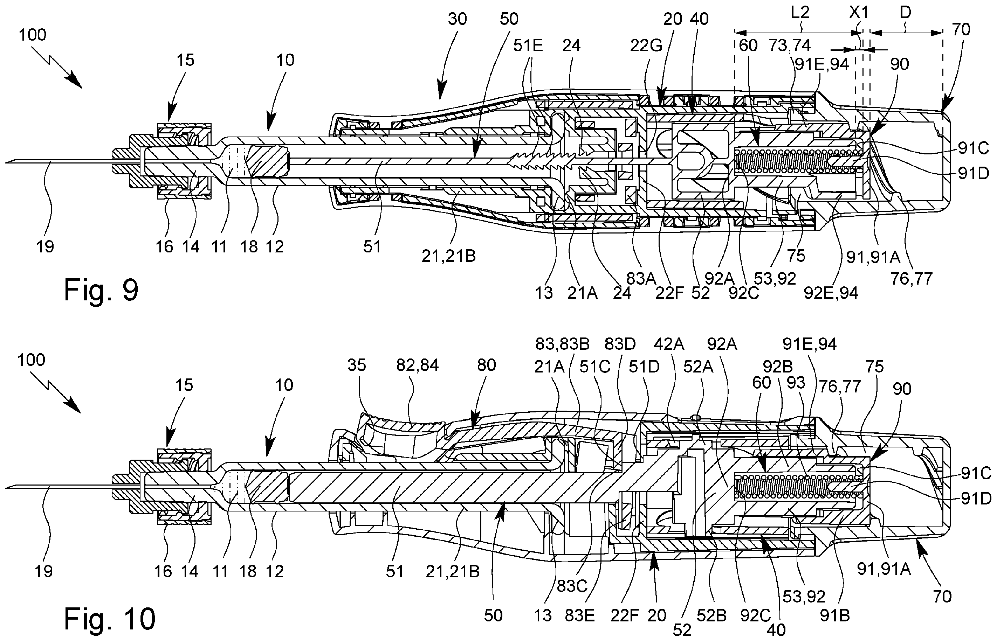

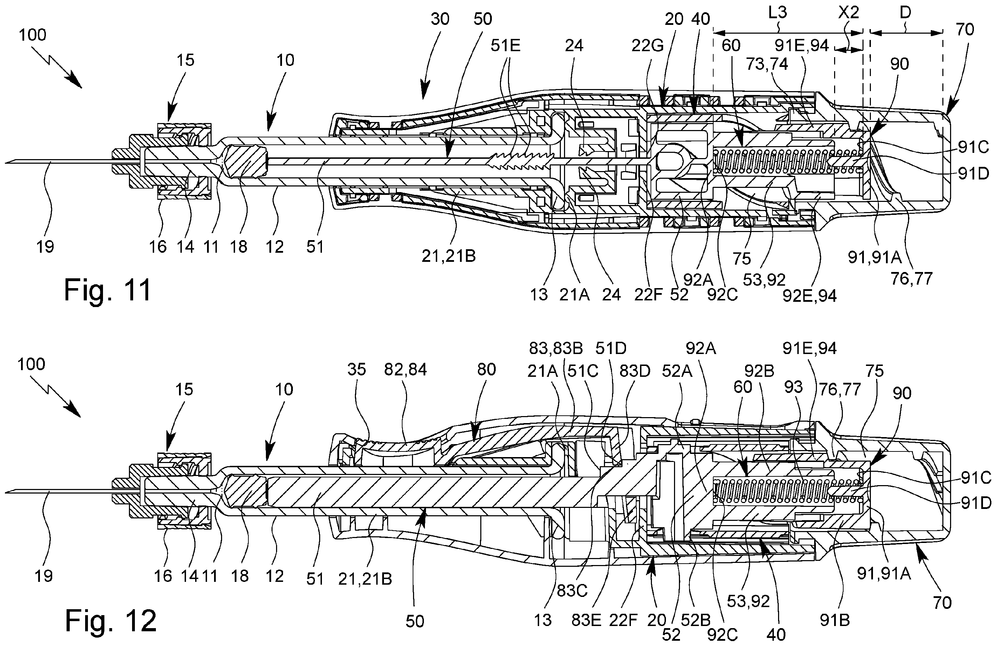

Figs. 5 and 6 show the administration device 100 in the initial position/state, i.e. before actuating the first actuation part 70. Figs. 7 and 8 show the administration device 100 in the releasing position, i.e. after actuating the first actuation part 70. Figs. 9 and 10 show the administration device 100 in the administration position/state, i.e. after priming and/or immediately before actuating the second actuation part 80. Figs. 11 and 12 show the administration device 100 in the final position/state, i.e. after actuating the second actuation part 80.

The initial position is preferably the (axial) position of the main unit 50, the stopper 18, the drive spring 60 and/or the primer 90 relative to the rack 20 and/or the container 10 before priming of the administration device 100 and/or before actuating the first actuation part 70 and/or in the initial state of the administration device 100.

In the initial state of the administration device 100 and/or when the main unit 50, the stopper 18, the drive spring 60 and/or the primer 90 are/is in the initial position, both the first actuation part 70 and the second actuation part 80 are unactuated.

It is preferably not possible to (directly) actuate the second actuation part 80 and/or to (directly) administer the fluid 11 in the initial state of the administration device 100 and/or when the main unit 50, the stopper 18, the drive spring 60 and/or the primer 90 are/is in the initial position.

In the initial position/state, the primer 90, in particular the first primer part 91 and/or the second primer part 92, and/or the end part 53 of the main unit 50 is/are preferably completely arranged within the first actuation part 70, in particular its receptacle 75.

Preferably, the bottom 91A of the first primer part 91 (axially) abuts/rests on the axial end of the first actuation part 70 in the initial state/position.

The (axial) length of the drive spring 60 preferably corresponds to the (axial) length of the spring chamber 93, i.e. the (axial) distance between the bottom 91A / spring seat 91C of the first primer part 91 and the bottom 92A / spring seat 92C of the second primer part 92.

In the initial position/state, the drive spring 60 is (most) tensioned/compressed, the spring chamber 93 is most compressed and/or the length of the drive spring 60 and/or the spring chamber 93 is minimal - compared to the other positions/states - and/or corresponds to a first and/or minimum length L1 (see Fig. 5), preferably wherein the first/minimum length L1 is greater than 10 mm or 15 mm and/or smaller than 30 mm or 25 mm.

As already mentioned, the drive spring 60 preferably moves, in particular pushes, the first primer part 91 and the second primer part 92 / the main unit 50 apart from each other.

The first actuation part 70 preferably comprises or forms a securing element 77, preferably wherein the securing element 77 is configured to prevent releasing of the drive spring 60 and/or a movement of the second primer part 92 / the main unit 50 relative to the first primer part 91 in the initial position/state and/or during actuating the first actuation part 70 and/or during movement of the stopper 18, the main unit 50, the drive spring 60 and/or the primer 90 from the initial position to the releasing position.

The securing element 77 is preferably embodied as a protrusion/ridge that extends helically into the receptacle 75 of the first actuation part 70.

Preferably, the securing element 77 comprises or forms the screw thread 76 of the first actuation part 70 or vice versa. However, other constructional solutions are possible as well, in particular wherein the securing element 77 and the screw thread 76 are embodied as separate parts/elements.

The securing element 77 / the screw thread 76 is preferably adapted to hold the first primer part 91 and the second primer part 92 / the main unit 50 axially together, at least in the initial position/state, until reaching the releasing position and/or during actuating the first actuation part 70 and/or during movement of the stopper 18, the main unit 50, the drive spring 60 and/or the primer 90 from the initial position to the releasing position.

In particular, the securing element 77 / the screw thread 76 (axially) engages the thread part 92E of the second primer part 92 such that the second primer part 92, in particular the thread part 92E of the second primer part 92, cannot be pushed out of the first primer part 91, in particular its slot 91F, by means of the drive spring 60, at least in the initial position/state and/or until the releasing postion is reached and/or during actuating the first actuation part 70 and/or during movement of the stopper 18, the main unit 50, the drive spring 60 and/or the primer 90 from the initial position to the releasing position.

As already mentioned, it is preferably not possible to actuate the second actuation part 80 in the initial state/position, during actuating the first actuation part 70 and/or during movement of the stopper 18, the main unit 50, the drive spring 60 and/or the primer 90 from the initial position to the administration position.

Preferably, the main unit 50, in particular the plunger rod 51, (radially) blocks and/or limits the movement of the second actuation part 80 in the initial state/position, during actuating the first actuation part 70 and/or during movement of the stopper 18, the main unit 50, the drive spring 60 and/or the primer 90 from the initial position to the administration position.

The second actuation part 80, in particular the second end 83 thereof, mostly preferred the frame 83B, preferably comprises or forms a locking element 83E, preferably wherein the locking element 83E prevents actuation of the second actuation part 80 in the initial state/position and/or before the administration position/state is reached, as best seen in Fig. 6.

The locking element 83E preferably engages/abuts the main unit 50, in particular the plunger rod 51, radially, mostly preferred such that the second actuation part 80, in particular its button 84, cannot be actuated, in particular pressed down.

It is thus preferred that the position and/or angle of rotation of the second actuation part 80 in particular with respect to the pivot axis P, is at least essentially fixed and/or limited in the initial state/position and/or during movement of the stopper 18, the main unit 50, the drive spring 60 and/or the primer 90 from the initial position to the administration position by means of the frame 83B that engages/abuts the main unit 50, in particular the plunger rod 51, from both sides, in Fig. 6 from below and above.

In particular, the position and/or angle of rotation of the second actuation part 80 is at least essentially fixed and/or limited in the initial state/position and/or during movement of the stopper 18, the main unit 50, the drive spring 60 and/or the primer 90 from the initial position to the administration position by means of locking element 83E engaging/abutting the main unit 50, in particular the rod plunger 51, from one side, in Fig. 6 from below, and by means of the blocking element 83D engaging/abutting the main unit 50, in particular the plunger rod 51, from the other side, in Fig. 6 from above.

As best seen in Fig. 5, the return lock, in particular the return stop 51E of the main unit 50 and the return stop 24 of the rack 20, is/are preferably not (yet) engaged and/or snapped into place in the initial state/position.

By actuating, in particular rotating, the first actuation part 70, the stopper 18, the main unit 50, the drive spring 60 and/or the primer 90 can be moved, in particular pushed, out of the initial position, in particular from the initial position to the releasing position and/or from the releasing position to the administration position.

Preferably, the primer 90, in particular the first primer part 91 and/or the second primer part 92, and/or the main unit 50 can be screwed out of the first actuation part 70 by actuating, in particular rotating, the first actuation part 70 due to the lead screw.

Until reaching the releasing position, the first primer part 91 and the second primer part 92 / the main unit 50 are preferably moved together and/or as a unit and/or without moving the first primer part 91 and the second primer part 92 / the main unit 50 relative to one another.

With other words, the length of the drive spring 60 and/or the spring chamber 94 remains preferably the same and/or is preferably not changed until the releasing position is reached and/or as long as the securing element 77 / the screw thread 76 of the first actuation part 70 engages the primer 90, in particular the first primer part 91, mostly preferred its thread part 91E.

Fig. 7 and 8 show the administration device 100 in the releasing position, i.e. after actuating the first actuation part 70.

The releasing position is preferably the (axial) position of the main unit 50, the stopper 18, the drive spring 60 and/or the primer 90 relative to the rack 20 and/or the container 10 between the initial position and the administration position and/or after completely actuating the first actuation part 70 and/or (immediately) before reaching the administration position/state and/or (immediately) before completing priming.

By actuating, in particular rotating, the first actuation part 70, the stopper 18, the main unit 50, the drive spring 60 and/or the primer 90 are/is axially moved, in particular pushed, towards the outlet of the administration device 100 and/or the container 10 and/or away from the first actuation part 70, preferably by at least 5 mm or 8 mm and/or at most 30 mm or 20 mm, in particular by at least essentially 10 mm.

In particular, the distance D between the main unit 50 and/or the primer 90, in particular the first primer part 91, mostly preferred its bottom 91A, on the one hand and the first actuation part 70, in particular its bottom, on the other hand is increased.

In the releasing position and/or when reaching the releasing position, the drive spring 60 / its spring force/energy is preferably (automatically) at least partially expanded/released, such that the subsequent movement of the main unit 50, the second primer part 92 and/or the stopper 18 towards the administration position and/or towards the outlet of the administration device 100 is completed and/or supported by means of the drive spring 60 and/or such that the transition of the administration device 100 from the initial state to the administration state is completed and/or supported by means of the drive spring 60.

In the releasing position and/or when reaching the releasing position, the screw thread 76 / the securing element 77 and the second primer part 92, in particular its thread part 92E, are (axially) disengaged, mostly preferred such that the drive spring 60 is partially released and/or can partially extend and/or such that the second primer part 92, in particular its thread part 92E, can at least partially be pushed out of the first primer part 91, in particular the slot 91F, by means of the drive spring 60. In this way, priming is completed and/or the administration position is reached.

Thus, according to one aspect of the invention, priming and/or the movement of the main unit 50, the second primer part 92 and/or the stopper 18 during priming and/or from the initial position to the administration position is completed in a semi-manual/semi-automatic manner and/or both by manually, i.e. by actuating, in particular rotating, the first actuation part 70, and automatically, i.e. by at least partially expanding/releasing the drive spring 60 / its spring force/energy (upon reaching the releasing position and/or from the releasing position to the administration position).

As already mentioned, the first primer part 91 and the second primer part 92 engage one another and/or the second primer part 92 is preferably configured to constrain the first primer part 92 radially/rotationally and/or in a circumferential direction, preferably such that the first primer part 91 and the second primer part 92 cannot rotate relative to one another, in particular at least during priming and/or until reaching the administration position.

According to a preferred embodiment (not shown), the first primer part 91 and the second primer part 92 disengage one another and/or the second primer part 92 is preferably configured to release the first primer part 91 radially/rotationally and/or in a circumferential direction, preferably such that the first primer part 91 and the second primer part 92 can rotate relative to one another after primer and/or at least temporarily and/or when the administration position is reached.

Preferably, after priming and/or when the administration position is reached, the drive spring 60 / its spring force/energy is at least partially (further) expanded/released and/or the first primer part 91 is moved, in particular rotated, back into and/or towards the first actuation part 70 by means of the drive spring 60, mostly preferred until the first primer part 91 and second primer part 92 engage on another again and/or until the first primer part 91 hits a corresponding stop of the second primer part 92.

In this way, an audible/haptic feedback is provided that clearly indicates the end of priming and/or when the administration position is reached, in particular in addition to and/or distinguishable from the audible/haptic feedback of the return lock, mostly preferred such that the user/practitioner can clearly discern priming and/or when the administration position/state is reached.

By actuating, in particular rotating, the first actuation part 70, the main unit 50, in particular its plunger rod 51, is axially moved, in particular pushed, (further) through the bottom 22F of the frame 22 and/or (further) through the second end 83 and/or the aperture 83C and/or frame 83B of the second actuation part 80.

As best seen in Fig. 7, upon actuation of the first actuation part 70 and/or at least between the initial position and the releasing position and/or during priming, the return lock of the actuation mechanism is preferably activated and/or the return stop 24 of the rack 20 preferably (axially) engages the return stop 51E of the main unit 50.

Thus, upon actuation of the first actuation part 70 and/or at least between the initial position and the releasing position and/or during priming, a back-movement of the main unit 50, i.e. a movement of the main unit 50 in the direction out of the container 10 and/or towards the first actuation part 70, is preferably prevented or at least limited due to the return lock.

As best seen in Fig. 8, the position and/or angle of rotation of the second actuation part 80 is preferably (still) at least essentially fixed and/or limited in the releasing position by means of the frame 83B, in particular the locking element 83E and/or the blocking element 83D, (radially) engaging/abutting the main unit 50, in particular the plunger rod 51, from both sides, in Fig. 8 from below and above.

It is preferred that the second actuation part 80, in particular the blocking element 83D of the second actuation part 80, is axially spaced apart from the first stop 51C of the main unit 50 and/or does not axially engage/block the main unit 50, in particular the plunger rod 51, mostly preferred in the initial position/state and/or in the releasing position and/or during the entire priming process.

Preferably, the second actuation part 80, in particular the blocking element 83D of the second actuation part 80, axially engages/blocks the main unit 50, in particular the plunger rod 51, only after completing priming and/or administration and/or only in the administration position/state and/or in the final position/state, as will be explained in the following with reference to Fig. 9 and 10 and Fig. 11 and 12.

Fig. 9 and 10 show the administration device 100 in the administration position, i.e. after partially releasing the drive spring 60 and/or completing priming.

The administration position is preferably the (axial) position of the main unit 50, the stopper 18 and/or the primer 90 relative to the rack 20 and/or the container 10 (immediately) after completing the priming process and/or (immediately) after (completely) actuating, in particular rotating, the first actuation part 70 and/or (immediately) before administration is carried out and/or in the administration state of the administration device 100.

In the administration state of the administration device 100, the first actuation part 70 is (completely) actuated and the second actuation part 80 is (still) unactuated.

In particular, in the administration state of the administration device 100, the second actuation part 80 can be (directly) actuated, in particular depressed, and/or the fluid 11 can be administered to a patient without performing further steps apart from actuating the second actuation part 80.

As already mentioned, the drive spring 60 preferably moves, in particular pushes, the first primer part 91 and the second primer part 92 apart from each other.

After reaching the releasing position, the second primer part 92 / the main unit 50 is moved relative to, in particular pushed out of, the first primer part 91 and/or towards the container 10 into the administration position/state by means of the drive spring 60.

Thus, in the administration position/state, the drive spring 60 is preferably less tensioned/compressed and/or further extended and/or the spring chamber 93 is longer/less compressed compared to the initial position/state and/or the releasing position.

In the administration position/state, the length of the drive spring 60 and/or the spring chamber 93 is increased compared to the initial position/state and/or the releasing position, preferably by at least 0.1 mm or 0.3 mm and/or by at most 2 mm or 1 mm, in particular by at least essentially 0.5 mm.

Preferably, the length of the drive spring 60 and/or the spring chamber 93 in the administration position/state corresponds to a second and/or intermediate length L2 (see Fig. 9), preferably wherein the second/intermediate length L2 is greater than the first/minimum length L1, preferably by a difference X1 as indicated in Fig. 9 and/or by at least 0.1 mm or 0.3 mm and/or by at most 2 mm or 1 mm, in particular by at least essentially 0.5 mm.

With other words, when/after reaching the releasing position, the drive spring 60 preferably pushes the main unit 50, in particular the plunger rod 51, further into the container 10, preferably by at least 0.1 mm or 0.3 mm and/or by at most 2 mm or 1 mm, in particular by at least essentially 0.5 mm, and/or until the main unit 50, in particular the plunger rod 51, mostly preferred the first stop 51C, (axially) abuts/hits the frame 83B and/or the blocking element 83D of the second actuation part 80, but seen in Fig. 10.

It is thus preferred that in the administration position, the second actuation part 80, in particular the frame 83B and/or the blocking element 83D of the second actuation part 80, (axially) engages the main unit 50, in particular the plunger rod 51, mostly preferred the first stop 51C thereof, thereby blocking further movement of the main unit 50 into the container 10.

Further releasing/extension of the drive spring 60 / its spring force/energy and/or movement of the main unit 50, in particular the plunger rod 51, into the container 10, is prevented by means of the second actuation part 80, in particular the frame 83B and/or the blocking element 83D of the second actuation part 80, that engages the main unit 50, in particular the plunger rod 51, mostly preferred the first stop 51C thereof, axially and/or in a form-fitting manner.

As already mentioned, the second actuation part 80 can be directly actuated in the administration position/state.

Preferably due to the (stepped) rear portion 51B of the plunger rod 51, the locking element 83E of the second actuation part 80 preferably no longer (radially) engages/abuts the plunger rod 51 in the administration position/state.

Preferably, the frame 83B and/or the locking element 83E of the second actuation part 80 on the one hand and the plunger rod 51 of the main unit 50, in particular the rear portion 51B thereof, on the other hand, are radially spaced apart from each other, in particular such that the second actuation part 80, in particular its button 84, can be actuated, in particular depressed, in the administration position/state, as best seen in Fig. 10.

With other words, the plunger rod 51 preferably does not block the second actuation part 80 from being actuated, in particular depressed, in the administration position/state.

By actuating the second actuation part 80, in particular by depressing the button 84, the administration of the fluid 11 can be carried out and/or the main unit 50 and/or the stopper 18 can be moved to the final position.

By actuating, in particular depressing, the second actuation part 80, in particular its button 84, the second actuation part 80, in particular its second end 83, is pivoted about the pivot axis P and/or at least essentially moved relative to and/or away from the main unit 50, in particular such that the blocking element 83D (axially) disengages the main unit 50, in particular its first stop 51C, and/or such that the drive spring 60 / its spring force/energy is (again) released and/or can (further) expand and/or move the main unit 50, in particular its plunger rod 51, further into the container 10.

The second actuation part 80 can be actuated, in particular depressed, preferably until the locking element 83E (again) radially abuts/engages the main unit 50, in particular the plunger rod 51. Thus, in the final position/state, the locking element 83E preferably (again) radially abuts/engages the main unit 50, in particular the plunger rod 51, thereby in particular preventing further depression of the second actuation part 80.

Fig. 11 and 12 show the administration device 100 in the final position/state, i.e. after actuating the second actuation part 80.

The final position is preferably the (axial) position of the main unit 50 and/or the stopper 18 relative to the rack 20 and/or the container 10 (immediately) after administration and/or after (completely) actuating the second actuation part 80 and/or in the final state of the administration device 100.

In the final state of the administration device 100 and/or when the main unit 50 and/or the stopper 18 are/is in the final position, the administration of the fluid 11 has been carried out, the second actuation part 80 is (completely) actuated and/or the drive spring 60 is at least partially (further) released.

In the final state/position, the main unit 50, the stopper 18 and/or the second primer part 92 are/is arranged closer to the outlet of the administration device 100 and/or further away from the first actuation part 70 and/or the first primer part 91 and/or are/is inserted further into the container 10 compared to the initial state/position and/or administration state/position.

In the final position/state, the second primer part 92 / the main unit 50 is preferably further moved, in particular pushed, out of the first primer part 91 by means of the drive spring 60 compared to the administration state/position.

In the final position/state, the drive spring 60 is less tensioned/compressed and/or further extended and/or the spring chamber 93 is longer/less compressed compared to the initial position/state and/or the administration position/state.

In the final position/state, the length of the drive spring 60 and/or the spring chamber 93 is (further) increased compared to the administration position/state, preferably by at least 1 mm or 2 mm and/or by at most 10 mm or 8 mm, in particular by at least essentially 5 mm.

Preferably, the length of the drive spring 60 and/or the spring chamber 93 in the final position/state corresponds to a third and/or final length L3 (see Fig. 11), preferably wherein the third/final length L3 is greater than the first/minimum length L1 and/or the second/intermediate length L2, preferably by a difference X2 as indicated in Fig. 11 and/or by at least 1 mm or 2 mm and/or by at most 10 mm or 8 mm, in particular by at least essentially 5 mm.

With other words, upon actuating the second actuation part 80, the drive spring 60 preferably pushes the main unit 50, in particular the plunger rod 51, further into the container 10, preferably by at least 1 mm or 2 mm and/or by at most 10 mm or 8 mm, in particular by at least essentially 5 mm, and/or until the main unit 50, in particular the plunger rod 51, mostly preferred the second stop 51D, (axially) abuts/hits the frame 83B and/or the blocking element 83D of the second actuation part 80, in particular thereby preventing further movement of the main unit 50 into the container 10.

Thus, according to one aspect of the present invention, the drive spring 60 / its spring force/energy is at least partially expandable/releasable by actuating, in particular rotating, the first actuation part 70 and at least partially expandable/releasable by actuating, in particular depressing, the second actuation part 80.

Thus, the energy stored in the pretensioned drive spring 60 is preferably released in two steps and/or by actuating the first actuation part 70 and, subsequently, the second actuation part 80.

In this way, the usability of the administration device 100 is improved and the transition from the initial state/position to the administration state/position and final state/position is facilitated.

According to another aspect of the present invention, the axial movement of the main unit 50 is limited by means of the second actuation part 80, in particular its blocking element 83D, both in the administration position/state and in the final position/state.

The use of the (same) blocking element 83D for stopping the axial movement of the main unit 50 after priming and administration improves the dose accuracy and, thereby, minimizes the requirements of/in the manufacturing processes.

In the final position/state, the position and/or angle of rotation of the second actuation part 80 is preferably (again) at least essentially fixed and/or limited by means of the frame 83B that engages/abuts the main unit 50, in particular the plunger rod 51, from both sides, in Fig. 12 from below and above.

In particular, the position and/or angle of rotation of the second actuation part 80 is preferably at least essentially fixed and/or limited in the final state/position by means of locking element 83E engaging/abutting the main unit 50, in particular the rod plunger 51, from one side, in Fig. 12 from below, and by means of the blocking element 83D engaging/abutting the main unit 50, in particular the plunger rod 51, from the other side, in Fig. 12 from above.

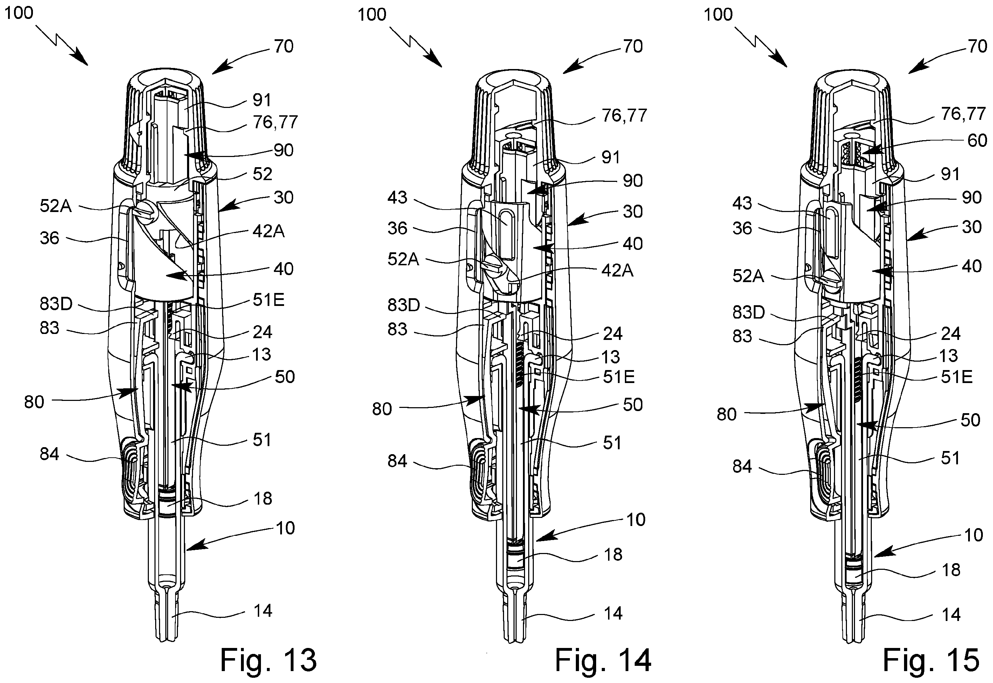

Referring now to Figs. 13 to 15, the mechanical interaction between the main unit 50 and the indicator part 40 will be described.

Fig. 13 is a perspective partial section of the administration device 100 in the initial state. Fig. 14 is a perspective partial section of the administration device 100 in the administration state. Fig. 15 is a perspective partial section of the administration device 100 in the final state.

As already mentioned, the main unit 50, in particular its intermediate part 52, mostly preferred the pin 52A/52B, preferably radially protrudes into and/or axially engages the indicator part 40, in particular its axial opening 41A/41B and/or helical slot/surface 42A/42B, mostly preferred only after reaching the administration position/state and/or during the movement from the administration position/state to the final position/state.

An axial movement of the main unit 50, in particular its pin 52A/52B, mostly preferred upon reaching the administration position, thus preferably causes a rotation of the indicator part 40, in particular around and/or relative to the main unit 50, in particular its intermediate part 52. In this way, the rotation angle of the indicator part 40 correlates to the axial position of the main unit 50.

It is preferred that both the indicating means of the main unit 50, in particular the pin 52A/52B, and the indicator part 40, in particular its indicating means 43, is visible from the outside and/or through the casing window 36.

Preferably, the indicating means of the main unit 50, in particular the pin 52A/52B, axially moves within the preferably elongated casing window 36, in particular from one end to the other end of the casing window 36, in Fig. 13 to 15 top down.

The position of the indicating means of the main unit 50, in particular the pin 52A/52B, within the casing window 36 thus directly indicates the axial position of the main unit 50 and/or the stopper 18, in particular the initial position, the releasing position, the administration position and/or the final position.

The casing 30, in particular the casing window 36, might be equipped with a label, a mark or the like corresponding to the initial position, the releasing position, the administration position and/or the final position, such that the position of the indicating means of the main unit 50, in particular the pin 52A/52B, relative to the label, the mark or the like indicates the position of the main unit 50 and/or stopper 18.

The indicator part 40, in particular its indicating means 43, preferably moves substantially transversely relative to the longitudinal extension of the (elongated) casing window 36, thereby preferably indicating the state of the administration device 100 and/or the axial position of the main unit 50 and/or the stopper 18, in particular the initial position, the releasing position, the administration position and/or the final position.

Mostly preferred, the indicator part 40, in particular its indicating means 43, is adapted to indicate the state of the administration device 100 and/or the axial position of the main unit 50 and/or the stopper 18, in particuar when the final state/position is reached.

In the present embodiment, the inidicating means 43 is embodied as an elongated mark, preferably of contrasting color, that moves substantially transversely into the casing window 36 when the final position/state is reached.

In this way, the usability is improved and/or the risk of misuse due to a possible misunderstanding of the status of the administration device 100 is decreased.

Further, the indicator part 40 restricts the visibility into the administration device 100, in particular through the casing window 36, thereby focusing the user's / practitioner's attention to the indicator part 40 and/or the indicating means of the main unit 50, in particular its pin 52A/52B.

The preferred method for operating the administration device 100 preferably comprises at least one of the following steps:
(manually) actuating, in particular rotating, the first actuation part 70, thereby axially moving the main unit 50, the stopper 18, the drive spring 60 and/or the primer 90 relative to the rack 20 and/or the container 10, in particular by means of a leadscrew of the actuation mechanism, mostly preferred by means of the screw thread 94 of the primer 90 interacting with the screw thread 76 of the first actuation part 70, and/or
(manually) actuating, in particular rotating, the first actuation part 70, in particular until the releasing position of the main unit 50 is reached, thereby releasing the second primer part 92, in particular its thread part 92E, and/or partially extending/releasing the drive spring 60 / its spring force, in particular such that the first primer part 91 and the second primer part 92 are moved apart from one another by means of the drive spring 60 and/or such that the end of the priming is completed automatically and/or by means of the spring force/energy of the drive spring 60, and/or
(manually) actuating, in particular depressing, the second actuation part 80, in particular its button 84, thereby (axially) disengaging the blocking element 83D of the second actuation part 80 from the main unit 50 and/or (again) at least partially (further) extending/releasing the drive spring 60 / its spring force, in particular such that the main unit 50 and/or the stopper 18 are moved from the administration position to the final position automatically and/or by means of the spring force/energy of the drive spring 60.

As already mentioned, the administration device 100 may be adapted for ophthalmic injections. In one embodiment, the administration device 100 is suitable for ophthalmic injections, and as such has a suitably small volume.

The administration device 100, in particular the container 10, may also be silicone free, or substantially silicone free, or may comprise a low level of silicone as lubricant. In one embodiment, the administration device 100 and/or the container 10 may meet the requirements of USP789.

The administration device 100, in particular the container 10, may be filled with any suitable injectable fluid/liquid/medication 11.

In one embodiment the administration device 100, in particular the container 10, is filled with fluid 11, in particular an injectable medicament, comprising an active suitable for the treatment of an ocular disease. Examples of such ocular diseases include choroidal neovascularisation, age-related macular degeneration (AMD, both wet and dry forms), macular edema secondary to retinal vein occlusion (RVO) including both branch RVO (bRVO) and central RVO (cRVO), choroidal neovascularisation secondary to pathologic myopia (PM), diabetic macular edema (DME), diabetic macular ischemia (DMI), geographic atrophy, diabetic retinopathy, and proliferative retinopathy.

In one embodiment, the fluid 11, in particular the injectable medicament, comprises a biologic active ingredient. The biologic active ingredient may be an antibody (or fragment thereof) or a non-antibody protein.

In one embodiment, the fluid 11, in particular the injectable medicament, comprises a VEGF antagonist, a multi-specific anti-VEGF/Ang2 antibody or a fragment thereof, a multi-specific anti-VEGF/AngPT2 antibody or a fragment thereof, a multi-specific anti-VEGF/TrkB agonist, or a multi-specific anti-VEGF/APL-2 complement C3 inhibitor, such as ranibizumab, bevacizumab, aflibercept, brolucizumab, conbercept (KH902) and the related glycoform KH906, faricinab or pazopanib.

The invention is defined by the appended claims.

**List of Reference Signs:**

| | | | |
|---|---|---|---|
| 100 | administration device | 34 | second axial opening (casing) |
| | | 35 | radial opening (casing) |
| 10 | container | 36 | casing window |
| 11 | fluid | | |
| 12 | main body | 40 | indicator part |
| 13 | first axial end (container) | 41A | 1st axial opening (indicator part) |
| 14 | second axial end (container) | 41B | 2nd axial opening (indicator part) |
| 15 | lock | 42A | 1st helical slot |
| 16 | connector | 42B | 2nd helical slot |
| 17 | cap | 43 | indicating means |
| 18 | stopper | | |
| 19 | needle | 50 | main unit |
| | | 51 | plunger rod |
| 20 | rack | 51A | front portion |
| 21 | mount | 51B | rear portion |
| 21A | socket (rack) | 51C | first stop |
| 21B | retainer | 51D | second stop |
| 22 | frame (rack) | 51E | return stop (main unit) |
| 22A | receptacle (rack) | 52 | intermediate part |
| 22B | axial opening (rack) | 52A | first pin |
| 22C | retaining element | 52B | second pin |
| 22D | back-rotation stop (rack) | | |
| 22E | actuation stop | 53 | end part (main unit) |
| 22F | bottom (rack) | | |
| 22G | opening (rack) | 60 | drive spring |
| 22H | first recess | | |
| 22J | second recess | 70 | first actuation part |
| | | 71 | handle |
| 23 | pivot pin | 72 | collar |
| 24 | return stop (rack) | 73 | connector (1st actuation part) |
| 30 | casing | 74 | back-rotation stop part) (1st actuation |
| 31 | first casing part | 75 | receptacle (1st actuation part) |
| 32 | second casing part | 76 | screw thread |
| 33 | first axial opening (casing) | 77 | securing element |
| 80 | second actuation part | A | main axis |
| 81 | socket (2nd actuation part) | D | distance |
| 82 | first end (2nd actuation part) | L1 | first length |
| 83 | second end (2nd actuation part) | L2 | second length |
| 83A | actuation spring | L3 | thrid length |
| 83B | frame (2nd actuation part) | P | pivot axis |
| 83C | aperture | R | rotation axis |
| 83D | blocking element | X1 | difference |
| 83E | locking element | X2 | difference |
| 84 | button | | |
| 85 | spacer | | |
| 90 | primer | | |
| 91 | first primer part | | |
| 91A | bottom (1st primer part) | | |
| 91B | wall (1st primer part) | | |
| 91C | spring seat (1st primer part) | | |
| 91D | spring pin (1st primer part) | | |
| 91E | thread part (1st primer part) | | |
| 91F | slot (1st primer part) | | |
| 91G | groove (1st primer part) | | |
| 92 | second primer part | | |
| 92A | bottom (2nd primer part) | | |
| 92B | wall (2nd primer part) | | |
| 92C | spring seat (2nd primer part) | | |
| 92E | thread part (2nd primer part) | | |
| 92G | ridge (2nd primer part) | | |
| 93 | spring chamber | | |
| 94 | screw thread | | |

## Claims

1. Administration device (100) for in particular intravitreal administration of a fluid (11), the administration device (100) comprising:
a rack (20), wherein a container (10) containing the fluid (11) can be held by the rack (20), and
an actuation mechanism comprising a main unit (50) acting on a stopper (18) movably arranged within the container (10), a compressed drive spring (60) acting on the main unit (50), a first actuation part (70) and a second actuation part (80),
wherein the first actuation part (70) and the second actuation part (80) are movably attached to the rack (20), the first actuation part (70) being axially secured to the rack (20) and rotatable relative to the rack (20),
**characterized,**
**in that** the first actuation part (70) can be actuated by manually rotating the first actuation part (70) relative to the rack (20) in order to move the main unit (50) and the drive spring (60) together from an initial position to a releasing position, wherein the actuation mechanism comprises a securing element (77) configured to prevent releasing/expansion of the drive spring (60) during movement of the main unit (50) and the drive spring (60) from the initial position to the releasing position and to at least partially release/expand the drive spring (60) when the releasing position is reached such that the main unit (50) is moved from the releasing position to an administration position by means of the drive spring (60), and
**in that** the second actuation part (80) comprises a blocking element (83D) configured to axially block the main unit (50) when the administration position is reached, wherein the second actuation part (80) can be actuated in order to at least partially release/expand the drive spring (60) and to move the main unit (50) from the administration position to a final position.

2. Administration device according to claim 1, **characterized in that** the actuation mechanism is configured to move the main unit (50) from the initial position to the administration position during priming of the administration device (100) and/or by actuating the first actuation part (70), and from the administration position to the final position during subsequent administration of the fluid (11) and/or by actuating the second actuation part (80).

3. Administration device according to any of the preceding claims, **characterized in that** the actuation mechanism comprises a primer (90) axially movable relative to the rack (20), wherein the primer (90) comprises a first primer part (91) and a second primer part (92), preferably wherein the first primer part (91) and the second primer part (92) are axially releasably connected to one another and/or wherein the second primer part (92) is connected to or formed by the main unit (50), in particular an end part (53) thereof.

4. Administration device according to claim 3, **characterized in that** the drive spring (60) is arranged at least partially within the primer (90) and/or **in that** the primer (90), in particular the first primer part (91), the second primer part (92) and/or the drive spring (60), are/is arranged at least partially within the first actuation part (70), at least in the initial state of the administration device (100), and/or **in that** the drive spring (60) is configured to move the first primer part (91) and the second primer (92) apart from each other.

5. Administration device according to claim 3 or 4, **characterized in that** the actuation mechanism comprises a leadscrew, wherein the first actuation part (70) and the primer (90), in particular the first primer part (91) and the second primer part (92), movably interact with one another by means of the leadscrew and/or wherein the first actuation part (70) is rotatable in order to axially move the primer (90), in particular the first primer part (91) and the second primer part (92), and/or the drive spring (60), in particular together and/or as a unit.

6. Administration device according to any of the claims 3 to 5, **characterized in that** the primer (90), in particular the first primer part (91) and the second primer part (92), comprises/comprise an in particular external screw thread (94) and **in that** the first actuation part (70) comprises an in particular internal screw thread (76), the screw thread (94) of the primer (90) and the screw thread (76) of the first actuation part (70) interacting with one another.

7. Administration device according to any of the claims 3 to 6, **characterized in that** the first primer part (91) and the second primer part (92) engage one another rotationally such that the first primer part (91) and the second primer part (92) cannot rotate relative to one another, in particular at least until reaching the administration position.

8. Administration device according to any of the claims 3 to 7, **characterized in that** the first primer part (91) and the second primer part (92) disengage one another rotationally when the administration position is reached, in particular such that the first primer part (91) and the second primer part (92) can rotate relative to one another.

9. Administration device according to any of the claims 3 to 8, **characterized in that** when the administration position is reached, the first primer part (91) is moved, in particular rotated, back into and/or towards the first actuation part (70) by means of the drive spring (60), mostly preferred until the first primer part (91) and second primer part (92) engage on another again and/or until the first primer part (91) hits a corresponding stop of the second primer part (92).

10. Administration device according to any of the preceding claims, **characterized in that** the securing element (77) is embodied as an in particular internal screw thread (76) and/or holds the first primer part (91) and the second primer part (92) axially together only until the releasing position is reached and/or wherein the securing element (77) can be disengaged by actuating the first actuation part (70).

11. Administration device according to any of the preceding claims, **characterized in that** the blocking element (83D) is configured to axially block the main unit (50) after at least partially releasing/expanding the drive spring (60) by means of the second actuation part (80) and/or when the final position is reached.

12. Administration device according to any of the preceding claims, **characterized in that** the main unit (50), in particular a plunger rod (51) thereof, comprises or forms a first stop (51C) and/or a second stop (51D), wherein the blocking element (83D) is adapted to axially engage the first stop (51C) in the administration position and/or after completing priming and/or to axially engage the second stop (51D) in the final position and/or after completing administration.

13. Administration device according to any of the preceding claims, **characterized in that** the administration device (100) comprises an indicator part (40) indicating the state of the administration device (100) and/or the axial position of the main unit (50), wherein the indicator part (40) is rotatable by the axial movement of the main unit (50).

14. Administration device according to claim 13, **characterized in that** the main unit (50), in particular an intermediate part (52) thereof, comprises at least one preferably arc-shaped indicating means, preferably wherein the indicating means extends over the indicator part (40) and/or is visible from the outside of the administration device (100), and/or **in that** the indicator part (40) comprises at least one helical or inclined slot and/or surface (42A, 42B).

15. Administration device according to claim 14, **characterized in that** main unit (50) radially protrudes into and/or axially engages the slot and/or surface (42A, 42B) such that an axial movement of the main unit (50) rotates the indicator part (40), preferably only during movment of the main unit (50) from the administration position to the final position.

## Patentansprüche

1. Verabreichungsvorrichtung (100) zur insbesondere intravitrealen Verabreichung eines Fluids (11), wobei die Verabreichungsvorrichtung (100) umfasst:
ein Gestell (20), wobei ein das Fluid (11) enthaltender Behälter (10) von dem Gestell (20) gehalten werden kann, und
einen Betätigungsmechanismus, der eine auf einen innerhalb des Behälters (10) beweglich angeordneten Stopfen (18) wirkende Haupteinheit (50), eine auf die Haupteinheit (50) wirkende komprimierte Antriebsfeder (60), ein erstes Betätigungsteil (70) und ein zweites Betätigungsteil (80) umfasst,
wobei das erste Betätigungsteil (70) und das zweite Betätigungsteil (80) beweglich an dem Gestell (20) angebracht sind, wobei das erste Betätigungsteil (70) axial an dem Gestell (20) befestigt und relativ zu dem Gestell (20) drehbar ist,
**dadurch gekennzeichnet,**
**dass** das erste Betätigungsteil (70) durch manuelles Drehen des ersten Betätigungsteils (70) relativ zu dem Gestell (20) betätigt werden kann, um die Haupteinheit (50) und die Antriebsfeder (60) zusammen aus einer Ausgangsposition in eine Freigabeposition zu bewegen, wobei der Betätigungsmechanismus ein Sicherungselement (77) umfasst, das dazu ausgelegt ist, ein Freigeben/Ausdehnen der Antriebsfeder (60) während einer Bewegung der Haupteinheit (50) und der Antriebsfeder (60) aus der Ausgangsposition in die Freigabeposition zu verhindern und die Antriebsfeder (60) bei Erreichen der Freigabeposition zumindest teilweise freizugeben/auszudehnen, so dass die Haupteinheit (50) mittels der Antriebsfeder (60) aus der Freigabeposition in eine Verabreichungsposition bewegt wird, und
**dass** das zweite Betätigungsteil (80) ein Blockierelement (83D) umfasst, das dazu ausgelegt ist, die Haupteinheit (50) bei Erreichen der Verabreichungsposition axial zu blockieren, wobei das zweite Betätigungsteil (80) betätigt werden kann, um die Antriebsfeder (60) zumindest teilweise freizugeben/auszudehnen und die Haupteinheit (50) aus der Verabreichungsposition in eine Endposition zu bewegen.

2. Verabreichungsvorrichtung nach Anspruch 1, **dadurch gekennzeichnet, dass** der Betätigungsmechanismus dazu ausgelegt ist, die Haupteinheit (50) während eines Primens der Verabreichungsvorrichtung (100) und/oder durch Betätigen des ersten Betätigungsteils (70) aus der Ausgangsposition in die Verabreichungsposition und während einer nachfolgenden Verabreichung des Fluids (11) und/oder durch Betätigen des zweiten Betätigungsteils (80) aus der Verabreichungsposition in die Endposition zu bewegen.

3. Verabreichungsvorrichtung nach einem der vorhergehenden Ansprüche, **dadurch gekennzeichnet, dass** der Betätigungsmechanismus einen relativ zu dem Gestell (20) axial beweglichen Primer (90) umfasst, wobei der Primer (90) ein erstes Primerteil (91) und ein zweites Primerteil (92) umfasst, wobei vorzugsweise das erste Primerteil (91) und das zweite Primerteil (92) axial lösbar miteinander verbunden sind und/oder wobei das zweite Primerteil (92) mit der Haupteinheit (50), insbesondere einem Endteil (53) davon, verbunden oder durch diese gebildet ist.

4. Verabreichungsvorrichtung nach Anspruch 3, **dadurch gekennzeichnet, dass** die Antriebsfeder (60) zumindest teilweise innerhalb des Primers (90) angeordnet ist und/oder dass der Primer (90), insbesondere das erste Primerteil (91), das zweite Primerteil (92) und/oder die Antriebsfeder (60), zumindest im Ausgangszustand der Verabreichungsvorrichtung (100) zumindest teilweise innerhalb des ersten Betätigungsteils (70) angeordnet ist und/oder dass die Antriebsfeder (60) dazu ausgelegt ist, das erste Primerteil (91) und das zweite Primerteil (92) auseinander zu bewegen.

5. Verabreichungsvorrichtung nach Anspruch 3 oder 4, **dadurch gekennzeichnet, dass** der Betätigungsmechanismus eine Gewindespindel umfasst, wobei das erste Betätigungsteil (70) und der Primer (90), insbesondere das erste Primerteil (91) und das zweite Primerteil (92), mittels der Gewindespindel beweglich miteinander zusammenwirken und/oder wobei das erste Betätigungsteil (70) drehbar ist, um den Primer (90), insbesondere das erste Primerteil (91) und das zweite Primerteil (92), und/oder die Antriebsfeder (60), insbesondere gemeinsam und/oder als Einheit, axial zu bewegen.

6. Verabreichungsvorrichtung nach einem der Ansprüche 3 bis 5, **dadurch gekennzeichnet, dass** der Primer (90), insbesondere das erste Primerteil (91) und das zweite Primerteil (92), ein insbesondere äußeres Schraubgewinde (94) umfasst und dass das erste Betätigungsteil (70) ein insbesondere inneres Schraubgewinde (76) umfasst, wobei das Schraubgewinde (94) des Primers (90) und das Schraubgewinde (76) des ersten Betätigungsteils (70) miteinander zusammenwirken.

7. Verabreichungsvorrichtung nach einem der Ansprüche 3 bis 6, **dadurch gekennzeichnet, dass** das erste Primerteil (91) und das zweite Primerteil (92) derart miteinander in Dreheingriff stehen, dass sich das erste Primerteil (91) und das zweite Primerteil (92), insbesondere zumindest bis zum Erreichen der Verabreichungsposition, nicht relativ zueinander drehen können.

8. Verabreichungsvorrichtung nach einem der Ansprüche 3 bis 7, **dadurch gekennzeichnet, dass** das erste Primerteil (91) und das zweite Primerteil (92) bei Erreichen der Verabreichungsposition miteinander in Dreheingriff stehen, insbesondere derart, dass sich das erste Primerteil (91) und das zweite Primerteil (92) relativ zueinander drehen können.

9. Verabreichungsvorrichtung nach einem der Ansprüche 3 bis 8, **dadurch gekennzeichnet, dass** das erste Primerteil (91) bei Erreichen der Verabreichungsposition mittels der Antriebsfeder (60) zurück in und/oder zu dem ersten Betätigungsteil (70) bewegt, insbesondere gedreht, wird, meist bevorzugt, bis das erste Primerteil (91) und das zweite Primerteil (92) wieder aufeinander eingreifen und/oder bis das erste Primerteil (91) auf einen entsprechenden Anschlag des zweiten Primerteils (92) trifft.

10. Verabreichungsvorrichtung nach einem der vorhergehenden Ansprüche, **dadurch gekennzeichnet, dass** das Sicherungselement (77) als ein insbesondere inneres Schraubgewinde (76) ausgebildet ist und/oder das erste Primerteil (91) und das zweite Primerteil (92) nur bis zum Erreichen der Freigabeposition axial zusammenhält und/oder wobei das Sicherungselement (77) durch Betätigen des ersten Betätigungsteils (70) außer Eingriff gebracht werden kann.

11. Verabreichungsvorrichtung nach einem der vorhergehenden Ansprüche, **dadurch gekennzeichnet, dass** das Blockierelement (83D) dazu ausgelegt ist, die Haupteinheit (50) nach zumindest teilweisem Freigeben/Ausdehnen der Antriebsfeder (60) mittels des zweiten Betätigungsteils (80) und/oder bei Erreichen der Endposition axial zu blockieren.

12. Verabreichungsvorrichtung nach einem der vorhergehenden Ansprüche, **dadurch gekennzeichnet, dass** die Haupteinheit (50), insbesondere eine Kolbenstange (51) davon, einen ersten Anschlag (51C) und/oder einen zweiten Anschlag (51D) umfasst oder bildet, wobei das Blockierelement (83D) dazu ausgelegt ist, in der Verabreichungsposition und/oder nach Abschluss des Primens axial mit dem ersten Anschlag (51C) in Eingriff zu stehen und/oder in der Endposition und/oder nach Abschluss der Verabreichung axial mit dem zweiten Anschlag (51D) in Eingriff zu stehen.

13. Verabreichungsvorrichtung nach einem der vorhergehenden Ansprüche, **dadurch gekennzeichnet, dass** die Verabreichungsvorrichtung (100) ein Anzeigeteil (40) umfasst, das den Zustand der Verabreichungsvorrichtung (100) und/oder die axiale Position der Haupteinheit (50) anzeigt, wobei das Anzeigeteil (40) durch die axiale Bewegung der Haupteinheit (50) drehbar ist.

14. Verabreichungsvorrichtung nach Anspruch 13, **dadurch gekennzeichnet, dass** die Haupteinheit (50), insbesondere ein Zwischenteil (52) davon, mindestens ein vorzugsweise bogenförmiges Anzeigemittel umfasst, wobei sich das Anzeigemittel vorzugsweise über das Anzeigeteil (40) erstreckt und/oder von außerhalb der Verabreichungsvorrichtung (100) sichtbar ist, und/oder dass das Anzeigeteil (40) mindestens einen spiralförmigen oder geneigten Schlitz und/oder eine geneigte Oberfläche (42A, 42B) umfasst.

15. Verabreichungsvorrichtung nach Anspruch 14, **dadurch gekennzeichnet, dass** die Haupteinheit (50) derart radial in den Schlitz und/oder die Oberfläche (42A, 42B) hineinragt und/oder axial in diese eingreift, dass eine axiale Bewegung der Haupteinheit (50) das Anzeigeteil (40) dreht, vorzugsweise nur während einer Bewegung der Haupteinheit (50) aus der Verabreichungsposition in die Endposition.

## Revendications

1. Dispositif d'administration (100) destiné en particulier à l'administration intravitréenne d'un fluide (11), le dispositif d'administration (100) comprenant :
une crémaillère (20), un récipient (10) contenant le fluide (11) pouvant être maintenu par la crémaillère (20), et
un mécanisme d'actionnement comprenant une unité principale (50) agissant sur un bouchon (18) disposé de manière mobile à l'intérieur du récipient (10), un ressort d'entraînement comprimé (60) agissant sur l'unité principale (50), une première partie d'actionnement (70) et une seconde partie d'actionnement (80),
la première partie d'actionnement (70) et la seconde partie d'actionnement (80) étant fixées de manière mobile à la crémaillère (20), la première partie d'actionnement (70) étant fixée axialement à la crémaillère (20) et pouvant tourner par rapport à la crémaillère (20),
**caractérisé en ce que**
la première partie d'actionnement (70) peut être actionnée en faisant tourner manuellement la première partie d'actionnement (70) par rapport à la crémaillère (20) afin de déplacer l'unité principale (50) et le ressort d'entraînement (60) ensemble d'une position initiale à une position de libération, le mécanisme d'actionnement comprenant un élément de fixation (77) configuré pour empêcher la libération/l'expansion du ressort d'entraînement (60) pendant le déplacement de l'unité principale (50) et du ressort d'entraînement (60) de la position initiale à la position de libération et pour libérer/expanser au moins partiellement le ressort d'entraînement (60) lorsque la position de libération est atteinte de sorte que l'unité principale (50) soit déplacée de la position de libération à une position d'administration au moyen du ressort d'entraînement (60), et
la seconde partie d'actionnement (80) comprend un élément de blocage (83D) configuré pour bloquer axialement l'unité principale (50) lorsque la position d'administration est atteinte, la seconde partie d'actionnement (80) pouvant être actionnée afin de libérer/expanser au moins partiellement le ressort d'entraînement (60) et de déplacer l'unité principale (50) de la position d'administration à une position finale.

2. Dispositif d'administration selon la revendication 1, **caractérisé en ce que** le mécanisme d'actionnement est configuré pour déplacer l'unité principale (50) de la position initiale à la position d'administration pendant l'amorçage du dispositif d'administration (100) et/ou en actionnant la première partie d'actionnement (70), et de la position d'administration à la position finale pendant l'administration ultérieure du fluide (11) et/ou en actionnant la seconde partie d'actionnement (80).

3. Dispositif d'administration selon l'une quelconque des revendications précédentes, **caractérisé en ce que** le mécanisme d'actionnement comprend une amorce (90) mobile axialement par rapport à la crémaillère (20), l'amorce (90) comprenant une première partie d'amorce (91) et une seconde partie d'amorce (92), de préférence la première partie d'amorce (91) et la seconde partie d'amorce (92) étant connectées l'une à l'autre de manière axialement amovible et/ou la seconde partie d'amorce (92) étant connectée à ou formée par l'unité principale (50), en particulier une partie d'extrémité (53) de celle-ci.

4. Dispositif d'administration selon la revendication 3, **caractérisé en ce que** le ressort d'entraînement (60) est disposé au moins partiellement à l'intérieur de l'amorce (90) et/ou **en ce que** l'amorce (90), en particulier la première partie d'amorce (91), la seconde partie d'amorce (92) et/ou le ressort d'entraînement (60), est/sont disposé(s) au moins partiellement à l'intérieur de la première partie d'actionnement (70), au moins dans l'état initial du dispositif d'administration (100), et/ou **en ce que** le ressort d'entraînement (60) est configuré pour déplacer la première partie d'amorce (91) et la seconde amorce (92) à distance l'une de l'autre.

5. Dispositif d'administration selon la revendication 3 ou 4, **caractérisé en ce que** le mécanisme d'actionnement comprend une vis-mère, la première partie d'actionnement (70) et l'amorce (90), en particulier la première partie d'amorce (91) et la seconde partie d'amorce (92), interagissant de manière mobile l'une avec l'autre au moyen de la vis-mère et/ou la première partie d'actionnement (70) pouvant tourner afin de déplacer axialement l'amorce (90), en particulier la première partie d'amorce (91) et la seconde partie d'amorce (92), et/ou le ressort d'entraînement (60), en particulier ensemble et/ou sous forme d'unité.

6. Dispositif d'administration selon l'une quelconque des revendications 3 à 5, **caractérisé en ce que** l'amorce (90), en particulier la première partie d'amorce (91) et la seconde partie d'amorce (92), comprend/comprennent un filetage de vis (94) en particulier externe et **en ce que** la première partie d'actionnement (70) comprend un filetage de vis (76) en particulier interne, le filetage de vis (94) de l'amorce (90) et le filetage de vis (76) de la première partie d'actionnement (70) interagissant l'un avec l'autre.

7. Dispositif d'administration selon l'une quelconque des revendications 3 à 6, **caractérisé en ce que** la première partie d'amorce (91) et la seconde partie d'amorce (92) viennent en prise l'une avec l'autre de manière rotative de telle sorte que la première partie d'amorce (91) et la seconde partie d'amorce (92) ne peuvent pas tourner l'une par rapport à l'autre, en particulier au moins jusqu'à ce que la position d'administration soit atteinte.

8. Dispositif d'administration selon l'une quelconque des revendications 3 à 7, **caractérisé en ce que** la première partie d'amorce (91) et la seconde partie d'amorce (92) se dégagent l'une de l'autre de manière rotative lorsque la position d'administration est atteinte, en particulier de telle sorte que la première partie d'amorce (91) et la seconde partie d'amorce (92) peuvent tourner l'une par rapport à l'autre.

9. Dispositif d'administration selon l'une quelconque des revendications 3 à 8, **caractérisé en ce que** lorsque la position d'administration est atteinte, la première partie d'amorce (91) est déplacée, en particulier tournée, de nouveau dans et/ou vers la première partie d'actionnement (70) au moyen du ressort d'entraînement (60), de manière préférée entre toutes jusqu'à ce que la première partie d'amorce (91) et la seconde partie d'amorce (92) viennent en prise l'une avec l'autre à nouveau et/ou jusqu'à ce que la première partie d'amorce (91) vienne en butée contre une butée correspondante de la seconde partie d'amorce (92).

10. Dispositif d'administration selon l'une quelconque des revendications précédentes, **caractérisé en ce que** l'élément de fixation (77) est réalisé sous forme de filetage de vis (76) en particulier interne et/ou maintient la première partie d'amorce (91) et la seconde partie d'amorce (92) ensemble axialement uniquement jusqu'à ce que la position de libération soit atteinte et/ou l'élément de fixation (77) pouvant être dégagé en actionnant la première partie d'actionnement (70).

11. Dispositif d'administration selon l'une quelconque des revendications précédentes, **caractérisé en ce que** l'élément de blocage (83D) est configuré pour bloquer axialement l'unité principale (50) après la libération/l'expansion au moins partielle du ressort d'entraînement (60) au moyen de la seconde partie d'actionnement (80) et/ou lorsque la position finale est atteinte.

12. Dispositif d'administration selon l'une quelconque des revendications précédentes, **caractérisé en ce que** l'unité principale (50), en particulier une tige de piston (51) de celle-ci, comprend ou forme une première butée (51C) et/ou une seconde butée (51D), l'élément de blocage (83D) étant adapté pour venir en prise axialement avec la première butée (51C) dans la position d'administration et/ou après l'amorçage complet et/ou pour venir en prise axialement avec la seconde butée (51D) dans la position finale et/ou après l'administration complète.

13. Dispositif d'administration selon l'une quelconque des revendications précédentes, **caractérisé en ce que** le dispositif d'administration (100) comprend une partie d'indicateur (40) indiquant l'état du dispositif d'administration (100) et/ou la position axiale de l'unité principale (50), la partie d'indicateur (40) pouvant tourner par le mouvement axial de l'unité principale (50).

14. Dispositif d'administration selon la revendication 13, **caractérisé en ce que** l'unité principale (50), en particulier une partie intermédiaire (52) de celle-ci, comprend au moins un moyen d'indication de préférence en forme d'arc, le moyen d'indication s'étendant de préférence sur la partie d'indicateur (40) et/ou étant visible depuis l'extérieur du dispositif d'administration (100), et/ou **en ce que** la partie d'indicateur (40) comprend au moins une fente et/ou surface hélicoïdale ou inclinée (42A, 42B).

15. Dispositif d'administration selon la revendication 14, **caractérisé en ce que** l'unité principale (50) fait saillie radialement dans et/ou vient en prise axialement avec la fente et/ou surface (42A, 42B) de telle sorte qu'un mouvement axial de l'unité principale (50) fasse tourner la partie d'indicateur (40), de préférence uniquement pendant le déplacement de l'unité principale (50) de la position d'administration à la position finale.
